# EUROPEAN PATENT APPLICATION

(11) **EP 4 285 898 A1**
(43) Date of publication of application: **06.12.2023**
(21) Application number: 22745238.0
(22) Date of filing: 25.01.2022
(51) Int. Cl.: A61K 31/4164, A61K 31/417, A61K 31/145, A61P 37/00

(54) **PATHWAY MODULATOR, PHARMACEUTICAL COMPOSITION HAVING SAME, USE THEREOF, AND THERAPEUTIC METHOD USING SAME**

(30) Priority: 26.01.2021 CN 202110103435
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: SUN, Qiaoling, Shanghai 201203 (CN); PAN, Ke, Shanghai 201203 (CN); DENG, Yijun, Shanghai 201203 (CN); CHEN, Yu, Shanghai 201203 (CN); LV, Jing, Shanghai 201203 (CN)
(74) Representative: Regimbeau
(86) International application number: PCT/CN2022/073849
(87) International publication number: WO 2022/161364

(57) **Abstract**

A pathway modulator, a pharmaceutical composition having same, use thereof, and a therapeutic method using same. The pathway modulator (comprising one or more of a dopamine β-hydroxylase inhibitor, a receptor agonist and a receptor antagonist) can inhibit development of autoimmune diseases by immunoregulation, thereby providing potential therapeutic drugs for the treatment of autoimmune diseases.

## Description

The present disclosure claims the priority of the Chinese patent application (application No. 2021101034359) filed on January 26, 2021.

### FIELD OF THE INVENTION

The present disclosure relates to a pathway modulator, a pharmaceutical composition comprisign the same, use thereof, and a therapeutic method using the same.

### BACKGROUND OF THE INVENTION

Dopamine β-hydroxylase (hereinafter referred to as DBH), also known as dopamine β-monooxygenase, is an enzyme encoded by the *DBH* gene in the human body (EC 1.14.17.1). DBH catalyzes the oxidation of dopamine by oxygen to generate norepinephrine and epinephrine as follows:

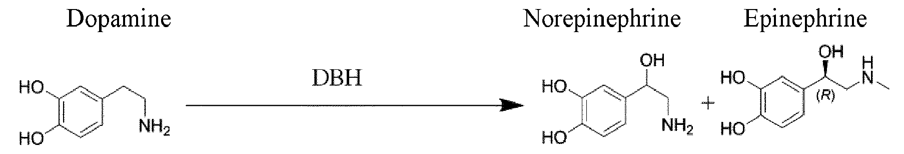

DBH is a copper-containing oxygenase of about 290 kDa consisting of four identical subunits, and its activity requires ascorbic acid as cofactor [1].

DBH is the only enzyme involved in the synthesis of small molecule membrane-bound neurotransmitters, making norepinephrine the only transmitter synthesized within vesicles. Norepinephrine is expressed in noradrenergic nerve endings of the central and peripheral nervous systems, as well as in chromaffin cells of the adrenal medulla.

DBH mainly contributes to the biosynthesis of trace amines and catecholamines, including epinephrine (or adrenaline), norepinephrine (or noradrenaline) and dopamine. In addition, it is involved in the metabolism of exogenous biomass related to these substances. For example, human DBH catalyzes the β-hydroxylation of amphetamine and p-hydroxyamphetamine, producing norephedrine and p-hydroxynorephedrine, respectively [2-4].

DBH is thought to be associated with decision thinking and drug addiction conditions, for example, alcoholism [5] and smoking [6], attention deficit hyperactivity disorder [7], schizophrenia [8] and Alzheimer's disease [9]. Lack of DBH is called dopamine β-hydroxylase deficiency.

At the end of the 20th century and the beginning of the 21st century, highly selective DBH inhibitors such as Nepicastat [10], Etamicastat [11], and Zamicastat [12] were successively developed, and their potential uses in hypertension (Pulmonary Arterial Hypertension), Congestive heart failure, Cocaine dependence or Post-traumatic stress disorder (PTSD), etc. have been further studied.

Nepicastat may be used in the treatment of congestive heart failure and has been shown to be well tolerated in patients [13]. Data from further clinical studies show that Nepicastat has no effect in the treatment of heart failure, but it is safe.

Nepicastat and analogues thereof (such as Etamicastat, Zamicastat) have potential use in the treatment of hypertension. Clinical trials evaluating the treatment of PTSD and cocaine dependence by Nepicastat have been completed. Studies have found that when Nepicastat is used in combination with cocaine, Nepicastat is safe and can inhibit the positive effects of cocaine. This result suggests that Nepicastat can be used as a drug therapy for cocaine dependence [14].

Compared with Nepicastat, Etamicastat and Zamicastat have lower levels of brain-blood-barrier penetration and can reduce the norepinephrine level in peripheral sympathetic innervation tissues, while having no effect on brain tissue of rats with spontaneous hypertension [15]. This result suggests that DBH inhibitors such as Etamicastat and Zamicastat can reduce adverse effects or complications in the central nervous system in the treatment of peripheral-related diseases. In a phase II clinical study, it was observed that Etamicastat (200 mg) reduced systolic and diastolic blood pressure in a dose-dependent manner after 10 days of treatment and showed good tolerance and safety [16]. Zamicastat (1200 mg) also showed relatively good safety when administered for 10 consecutive days (NCT02151994). A clinical trial of the efficacy of Zamicastat for pulmonary arterial hypertension is in progress (NCT04316143).

In a study as early as in 1998, the effect of DBH on immunoregulation was reported. Studies have found that in the absence of pathogens, dbh-/- mice have normal white blood cell counts, normal development of T and B cells, and these cells have normal functions *in vitro,* but dbh-/- mice are more susceptible to infection with pathogens (such as *Listeria monocytogenes* or *Mycobacterium tuberculosis*); meanwhile, the animals exhibit severe thymus degeneration and impaired T cell functions, including the production of Th1 cytokines. These results suggest that catecholamines are not required for normal physiologic development, but catecholamines play an important role in the immunoregulation of infection [17].

As part of the autonomic nerves, DBH penetrates directly and indirectly through the entire brain, tissues, blood vessels, and peripheral blood through noradrenergic fibers. In addition to noradrenergic fibers, its protein expression and localization also have certain tissue specificity. For example, DBH is expressed at certain levels in chromaffin cells of adrenal medulla, noradrenergic cells in locus coeruleus, liver tissues and intestinal tissues, among which DBH is most highly expressed in the adrenal medulla.

A study found that high expression of DBH in tissues or high levels of DBH secretion in peripheral blood may be associated with some neurological or endocrine diseases [18]. For example, high levels of DBH in peripheral blood may be associated with Alzheimer's disease, bipolar disorder, Huntington's disease, hypothyroidism and PTSD. Although levels of DBH in peripheral blood may be associated with neurological disorders, DBH inhibitors have not been shown to have a good efficacy in Alzheimer's disease and PTSD in clinical trials (see the CLINICALTRIALS database). Similarly, a study found that DBH expression in inflammatory tissues was upregulated in patients with autoimmune enteritis, but this study did not clarify their necessary connection [19].

According to the Autoimmune Association, there are more than 100 types of autoimmune diseases. In the clinic, the treatment of autoimmune diseases focuses on controlling symptoms with non-targeted drugs such as hormones and/or immunosuppressants, while bringing great and irreversible adverse effects and damage to the patient.

It can be seen that the search for a safe therapeutic agent capable of treating autoimmune diseases is an urgent technical problem in this field.

### SUMMARY OF THE INVENTION

The technical problems to be solved by the present disclosure include providing a pathway modulator, a pharmaceutical composition having the same, use thereof, and a method of treatment using the same.

The inventors have unexpectedly found that pathway modulators in prior art, including one or more of dopamine β-hydroxylase inhibitor (referred to as DBH inhibitors), receptor agonist and receptor antagonist, can inhibit the development and progression of autoimmune diseases by immunoregulation (e.g., in particular, ameliorating weight loss, improving DAI score, and returning colon density to normal), thereby providing potential therapeutic drugs for the treatment of autoimmune diseases.

The present disclosure solves the above technical problems through the following technical solutions:
The first aspect of the present disclosure relates to use of a pathway modulator in the preparation of a medicament for the treatment of autoimmune diseases; wherein, the pathway modulator is selected from the group consisting of DBH inhibitor, receptor agonist, receptor antagonist, and the combination thereof.

In the above use, the pathway modulator is preferably a DBH inhibitor.

In the above use, the DBH inhibitor can be a DBH inhibitor disclosed in the prior art or a future DBH inhibitor, and for example can be one or more of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof. The DBH inhibitor is preferably selected from the group consisting of Nepicastat, Etamicastat, Zamicastat, a pharmaceutically acceptable salt thereof, a prodrug thereof and the combination thereof. The DBH inhibitor is more preferably selected from the group consisting of Nepicastat, Etamicastat and Zamicastat. The use of one or more of the Nepicastat, Etamicastat and Zamicastat to treat autoimmune diseases will not cause the great and irreversible adverse effects and damage to patients brought by non-targeted drugs such as hormones and/or immunosuppressants, and makes it a safe therapeutic agent for the treatment of autoimmune diseases.

In the above use, the DBH inhibitor is preferably selected from the group consisting of Nepicastat, a pharmaceutically acceptable salt thereof, a prodrug thereof and the combination thereof. The DBH inhibitor is more preferably Nepicastat. Treatment of autoimmune diseases with Nepicastat can significantly reduce the weight loss of the patient and significantly reduce the colon density of the patient.

In the above use, as examples:
the pathway modulator is Nepicastat or a pharmaceutically acceptable salt thereof, and the unit dose is 10-100 mg/kg, for example 10, 15, 20, 25, 30, 35, 40, 45, 50, 55, 60, 65, 70, 75, 80, 85, 90, 95, 100 mg/kg, or the range between any two of the above values, preferably 20-50 mg/kg; or,
the pathway modulator is Etamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Zamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fusaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Disulfiram or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fumaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg.

In context, the unit dose can be an integer or a fraction. It should be understood that, for example, "10 to 100" is a concise expression, although not indicating each point value in the range, it is deemed to have been explicitly disclosed in the context.

The above technical solutions are used for the treatment of autoimmune diseases and can significantly reduce the weight loss of the patient and significantly reduce the colon density of the patient.

In the above use, the autoimmune diseases can be one or more of the autoimmune diseases disclosed in the prior art, for example can be selected from the group consisting of Achalasia, Addison's disease, Adult Still's disease, Agammaglobulinemia, Alopecia areata, Amyloidosis, Ankylosing spondylitis, Anti-GBM/Anti-TBM nephritis, Antiphospholipid syndrome, Autoimmune angioedema, Autoimmune dysautonomia, Autoimmune encephalomyelitis, Autoimmune hepatitis, Autoimmune inner ear disease (AIED), Autoimmune myocarditis, Autoimmune oophoritis, Autoimmune orchitis, Autoimmune pancreatitis, Autoimmune retinopathy, Autoimmune urticaria, Axonal & neuronal neuropathy (AMAN), Bal6 disease, Behcet's disease, Benign mucosal pemphigoid, Bullous pemphigoid, Castleman disease (CD), Celiac disease, Chagas disease, Chronic inflammatory demyelinating polyneuropathy (CIDP), Chronic recurrent multifocal osteomyelitis (CRMO), Eosinophilic Granulomatosis (EGPA), Cicatricial pemphigoid, Cogan's syndrome, Cold agglutinin disease, Congenital heart block, Coxsackie myocarditis, CREST syndrome, Crohn's disease, Dermatitis herpetiformis, Dermatomyositis, Devic's disease (neuromyelitis optica), Discoid lupus, Dressler's syndrome, Endometriosis, Eosinophilic esophagitis (EoE), Eosinophilic fasciitis, Erythema nodosum, Essential mixed cryoglobulinemia, Evans syndrome, Fibromyalgia, Fibrosing alveolitis, Giant cell arteritis, Giant cell myocarditis, Glomerulonephritis, Goodpasture's syndrome, Granulomatosis with Polyangiitis, Graves'disease, Guillain-Barre syndrome, Hashimoto's thyroiditis, Hemolytic anemia, Henoch-Schonlein purpura (HSP), Herpes gestationis or pemphigoid gestationis (PG), Hidradenitis Suppurativa (HS), Hypogammalglobulinemia, IgA Nephropathy, IgG4-related sclerosing disease, Immune thrombocytopenic purpura (ITP), Inclusion body myositis (IBM), Interstitial cystitis (IC), Juvenile arthritis, Juvenile diabetes Type 1, Juvenile myositis (JM), Kawasaki disease, Lambert-Eaton syndrome, Leukocytoclastic vasculitis, Lichen planus, Lichen sclerosus, Ligneous conjunctivitis, Linear IgA disease (LAD), Lupus, Lyme disease chronic, Meniere's disease, Microscopic polyangiitis (MPA), Mixed connective tissue disease (MCTD), Mooren's ulcer, Mucha-Habermann disease, Multifocal Motor Neuropathy (MMN or MMNCB), Multiple sclerosis, Myasthenia gravis, Myositis, Narcolepsy, Neonatal Lupus, Neutropenia, Ocular cicatricial pemphigoid, Optic neuritis, Palindromic rheumatism (PR), PANDAS, Paraneoplastic cerebellar degeneration (PCD), Paroxysmal nocturnal hemoglobinuria (PNH), Parry Romberg syndrome, Pars planitis, Parsonage-Turner syndrome, Pemphigus, Peripheral neuropathy, Perivenous encephalomyelitis, Pernicious anemia (PA), POEMS syndrome, Polyarteritis nodosa, Polyglandular syndromes type I, Polyglandular syndromes type II, Polyglandular syndromes type III, Polymyalgia rheumatica, Polymyositis, Postmyocardial infarction syndrome, Postpericardiotomy syndrome, Primary biliary cirrhosis, Primary sclerosing cholangitis, Progesterone dermatitis, Psoriasis, Psoriatic arthritis, Pure red cell aplasia (PRCA), Pyoderma gangrenosum, Raynaud's phenomenon, Reactive Arthritis, Reflex sympathetic dystrophy, Relapsing polychondritis, Restless legs syndrome (RLS), Retroperitoneal fibrosis, Rheumatic fever, Rheumatoid arthritis, Sarcoidosis, Schmidt syndrome, Scleritis, Scleroderma, Sjögren's syndrome, Sperm & testicular autoimmunity, Stiff person syndrome (SPS), Subacute bacterial endocarditis (SBE), Susac's syndrome, Sympathetic ophthalmia (SO), Takayasu's arteritis, Temporal arteritis/Giant cell arteritis, Thrombocytopenic purpura (TTP), Thyroid eye disease (TED), Tolosa-Hunt syndrome (THS), Transverse myelitis, Type 1 diabetes, Ulcerative colitis (UC), Undifferentiated connective tissue disease (UCTD), Uveitis, Vasculitis, Vitiligo and Vogt-Koyanagi-Harada Disease; preferably autoimmune colitis, neuromyelitis optica, rheumatoid arthritis, scleroderma, psoriasis, or uveitis.

The second aspect of the present disclosure relates to a pathway modulator for use in the treatment of autoimmune diseases; wherein, the pathway modulator is selected from the group consisting of DBH inhibitor, receptor agonist, receptor antagonist, and the combination thereof.

In the above pathway modulator, the pathway modulator is preferably a DBH inhibitor.

In the above pathway modulator, the DBH inhibitor can be a DBH inhibitor disclosed in the prior art, and for example can be one or more of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof. The DBH inhibitor is preferably selected from the group consisting of Nepicastat, Etamicastat, Zamicastat, a pharmaceutically acceptable salt thereof, a prodrug thereof and the combination thereof. The DBH inhibitor is more preferably selected from the group consisting of Nepicastat, Etamicastat and Zamicastat.

In the above pathway modulator, the DBH inhibitor is preferably selected from the group consisting of Nepicastat, a pharmaceutically acceptable salt thereof, a prodrug thereof and the combination thereof. The DBH inhibitor is more preferably Nepicastat.

In the above pathway modulator, as examples:
the pathway modulator is Nepicastat or a pharmaceutically acceptable salt thereof, and the unit dose is 10-100 mg/kg, for example 10, 15, 20, 25, 30, 35, 40, 45, 50, 55, 60, 65, 70, 75, 80, 85, 90, 95, 100 mg/kg, or the range between any two of the above values, preferably 20-50 mg/kg; or,
the pathway modulator is Etamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Zamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fusaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Disulfiram or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fumaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg.

In the above pathway modulator, the definition of the autoimmune diseases is as previously defined.

The third aspect of the present disclosure relates to a method for the treatment of autoimmune diseases, including the step of administering a therapeutically effective amount of pathway modulator to a subject; wherein, the pathway modulator is selected from the group consisting of DBH inhibitor, receptor agonist and receptor antagonist.

In the above method of treatment, the pathway modulator is preferably a DBH inhibitor.

In the above method of treatment, the DBH inhibitor can be a DBH inhibitor disclosed in the prior art, and for example can be one or more of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof. The DBH inhibitor is preferably selected from the group consisting of Nepicastat, Etamicastat, Zamicastat, a pharmaceutically acceptable salt thereof, a prodrug thereof and the combination thereof. The DBH inhibitor is more preferably selected from the group consisting of Nepicastat, Etamicastat and Zamicastat.

In the above method of treatment, the DBH inhibitor is preferably selected from the group consisting of Nepicastat, a pharmaceutically acceptable salt thereof, a prodrug thereof and the combination thereof. The DBH inhibitor is more preferably Nepicastat.

In the above method of treatment, the DBH inhibitor can be administered in combination with one or more selected from the group consisting of chemotherapeutic agent, targeted therapeutic agent, immunotherapy agent and anti-inflammatory agent.

In the above method of treatment, as examples:
the pathway modulator is Nepicastat or a pharmaceutically acceptable salt thereof, and the unit dose is 10-100 mg/kg, for example 10, 15, 20, 25, 30, 35, 40, 45, 50, 55, 60, 65, 70, 75, 80, 85, 90, 95, 100 mg/kg, or the range between any two of the above values, preferably 20-50 mg/kg; or,
the pathway modulator is Etamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Zamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fusaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Disulfiram or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fumaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg.

In the above method of treatment, the definition of the autoimmune diseases is as previously defined.

The fourth aspect of the present disclosure relates to a pharmaceutical composition for the treatment of autoimmune diseases, comprising:
- a pathway modulator, and
- a pharmaceutically acceptable carrier;
wherein, the pathway modulator is selected from the group consisting of DBH inhibitor, receptor agonist, receptor antagonist, and the combination thereof.

In the above pharmaceutical composition, the pathway modulator is preferably a DBH inhibitor.

In the above pharmaceutical composition, the DBH inhibitor can be a DBH inhibitor disclosed in the prior art, and for example can be one or more of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof. The DBH inhibitor is preferably selected from the group consisting of Nepicastat, Etamicastat, Zamicastat, a pharmaceutically acceptable salt thereof, a prodrug thereof and the combination thereof. The DBH inhibitor is more preferably selected from the group consisting of Nepicastat, Etamicastat and Zamicastat.

In the above pharmaceutical composition, the DBH inhibitor is preferably selected from the group consisting of Nepicastat, a pharmaceutically acceptable salt thereof, a prodrug thereof and the combination thereof. The DBH inhibitor is more preferably Nepicastat.

In the above pharmaceutical composition, as examples:
the pathway modulator is Nepicastat or a pharmaceutically acceptable salt thereof, and the unit dose is 10-100 mg/kg, for example 10, 15, 20, 25, 30, 35, 40, 45, 50, 55, 60, 65, 70, 75, 80, 85, 90, 95, 100 mg/kg, or the range between any two of the above values, preferably 20-50 mg/kg; or,
the pathway modulator is Etamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Zamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fusaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Disulfiram or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fumaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg.

In the above pharmaceutical composition, the definition of the autoimmune diseases is as previously defined.

The fifth aspect of the present disclosure relates to use of a pathway modulator in the preparation of a medicament; the medicament is used for one or more uses selected from the group consisting of: reducing the proportion of CD4+ T cells, increasing the proportion of regulatory T cells, increasing the proportion of CD8+ T cells, reducing the secretion of pro-inflammatory factors of CD4+ T cells, reducing the secretion of pro-inflammatory factors of CD8+ T cells, inhibiting the activation of B cells and inhibiting the activation of NK cells; reducing the content of lymphocytes, neutrophils and monocytes in the peripheral blood; reducing inflammatory cell infiltration and subdermal capillary hyperplasia in the dermis layer; ameliorating skin fibrosis; reducing the incidence of uveitis; ameliorating skin inflammation; improving stool form score, improving CW/CL, improving CW/BW, improving CW/CL/BW, inhibiting the increase of colon ulcer area, improving colon inflammatory cell infiltration score, improving tissue damage score; improving disease activity score, ameliorating hematochezia or occult blood. Wherein, the pathway modulator is selected from the group consisting of DBH inhibitor, receptor agonist, receptor antagonist, and the combination thereof.

In the above use, the pathway modulator is preferably a DBH inhibitor.

In the above use, the DBH inhibitor can be a DBH inhibitor disclosed in the prior art, and for example can be one or more of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof. The DBH inhibitor is preferably selected from the group consisting of Nepicastat, Etamicastat, Zamicastat, a pharmaceutically acceptable salt thereof, a prodrug thereof and the combination thereof. The DBH inhibitor is more preferably selected from the group consisting of Nepicastat, Etamicastat and Zamicastat.

In the above use, the DBH inhibitor is preferably selected from the group consisting of Nepicastat, a pharmaceutically acceptable salt thereof, a prodrug thereof and the combination thereof. The DBH inhibitor is more preferably Nepicastat.

In the above use, as examples:
the pathway modulator is Nepicastat or a pharmaceutically acceptable salt thereof, and the unit dose is 10-100 mg/kg, for example 10, 15, 20, 25, 30, 35, 40, 45, 50, 55, 60, 65, 70, 75, 80, 85, 90, 95, 100 mg/kg, or the range between any two of the above values, preferably 20-50 mg/kg; or,
the pathway modulator is Etamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Zamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fusaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Disulfiram or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fumaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg.

In the above use, the medicament is preferably used for one or more uses selected from the group consisting of: reducing the proportion of CD4+ T cells, increasing the proportion of regulatory T cells, reducing the secretion of pro-inflammatory factors of CD4+ T cells, inhibiting the activation of B cells and inhibiting the activation of NK cells.

In the above use, the pro-inflammatory factors secreted from CD4+ T cells are preferably one or more of IL-17A, IFN-γ and TNF-α.

In the above use, the pro-inflammatory factors secreted from CD8+ T cells are preferably IL-17A and/or TNF-α.

In the above use, the regulatory T cells are preferably CD25+FOXP3+ Treg cells.

In the above use, the B cells are preferably B220+ cells, more preferably CD69+B220+ B cells.

In the above use, the NK cells are preferably NK1.1+ cells, more preferably NK1.1+CD107a+ NK cells.

The sixth aspect of the present disclosure relates to a pathway regulator for use in one or more uses selected from the group consisting of: reducing the proportion of CD4+ T cells, increasing the proportion of regulatory T cells, increasing the proportion of CD8+ T cells, reducing the secretion of pro-inflammatory factors of CD4+ T cells, reducing the secretion of pro-inflammatory factors of CD8+ T cells, inhibiting the activation of B cells and inhibiting the activation of NK cells; reducing the content of lymphocytes, neutrophils and monocytes in the peripheral blood; reducing inflammatory cell infiltration and subdermal capillary hyperplasia in the dermis layer; ameliorating skin fibrosis; reducing the incidence of uveitis; ameliorating skin inflammation; improving stool form score, improving CW/CL, improving CW/BW, improving CW/CL/BW, inhibiting the increase of colon ulcer area, improving colon inflammatory cell infiltration score, improving tissue damage score; improving disease activity score, ameliorating hematochezia or occult blood. Wherein, the pathway modulator is selected from the group consisting of DBH inhibitor, receptor agonist, receptor antagonist, and the combination thereof.

In the above pathway modulators, the pathway modulator is preferably a DBH inhibitor.

In the above pathway modulators, the DBH inhibitor can be a DBH inhibitor disclosed in the prior art, and for example can be one or more of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof. The DBH inhibitor is preferably selected from the group consisting of Nepicastat, Etamicastat, Zamicastat, a pharmaceutically acceptable salt thereof, a prodrug thereof and the combination thereof. The DBH inhibitor is more preferably selected from the group consisting of Nepicastat, Etamicastat and Zamicastat.

In the above pathway modulators, the DBH inhibitor is preferably selected from the group consisting of Nepicastat, a pharmaceutically acceptable salt thereof, a prodrug thereof and the combination thereof. The DBH inhibitor is more preferably Nepicastat. In the above pathway modulators, as examples:
the pathway modulator is Nepicastat or a pharmaceutically acceptable salt thereof, and the unit dose is 10-100 mg/kg, for example 10, 15, 20, 25, 30, 35, 40, 45, 50, 55, 60, 65, 70, 75, 80, 85, 90, 95, 100 mg/kg, or the range between any two of the above values, preferably 20-50 mg/kg; or,
the pathway modulator is Etamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Zamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fusaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Disulfiram or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fumaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg.

In the above pathway modulators, the medicament is preferably for use in one or more uses selected from the group consisting of: reducing the proportion of CD4+ T cells, increasing the proportion of regulatory T cells, reducing the secretion of pro-inflammatory factors of CD4+ T cells, inhibiting the activation of B cells and inhibiting the activation of NK cells.

In the above pathway modulators, the pro-inflammatory factors secreted fromCD4+ T cells are preferably one or more of IL-17A, IFN-γ and TNF-α;

In the above pathway modulators, the pro-inflammatory factors secreted from CD8+ T cells are preferably IL-17A and/or TNF-α.

In the above pathway modulators, the regulatory T cells are preferably CD25+FOXP3+ Treg cells.

In the above pathway modulators, the B cells are preferably B220+ cells, more preferably CD69+B220+ B cells.

In the above pathway modulators, the NK cells are preferably NK1.1+ cells, more preferably NK1.1+CD107a+ NK cells.

The seventh aspect of the present disclosure relates to a method for the regulation of immune cell functions *in vivo* or *in vitro*, including the step of contacting an effective amount of pathway modulator with immune cells *in vivo* or *in vitro*, the immune cells are from a subject; wherein, the pathway modulator is selected from the group consisting of DBH inhibitor, receptor agonist, receptor antagonist, and the combination thereof;
the regulation of immune cell functions refers to one or more functions selected from the group consisting of: reducing the proportion of CD4+ T cells, increasing the proportion of regulatory T cells, increasing the proportion of CD8+ T cells, reducing the secretion of pro-inflammatory factors of CD4+ T cells, reducing the secretion of pro-inflammatory factors of CD8+ T cells, inhibiting the activation of B cells and inhibiting the activation of NK cells.

In the above method, the pathway modulator is preferably a DBH inhibitor.

In the above method, the DBH inhibitor can be a DBH inhibitor disclosed in the prior art, and for example can be one or more of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof. The DBH inhibitor is preferably selected from the group consisting of Nepicastat, Etamicastat, Zamicastat, a pharmaceutically acceptable salt thereof, a prodrug thereof and the combination thereof. The DBH inhibitor is more preferably selected from the group consisting of Nepicastat, Etamicastat and Zamicastat.

In the above method, the DBH inhibitor is preferably selected from the group consisting of Nepicastat, a pharmaceutically acceptable salt thereof, a prodrug thereof and the combination thereof. The DBH inhibitor is more preferably Nepicastat.

In the above method, as examples:
the pathway modulator is Nepicastat or a pharmaceutically acceptable salt thereof, and the unit dose is 10-100 mg/kg, for example 10, 15, 20, 25, 30, 35, 40, 45, 50, 55, 60, 65, 70, 75, 80, 85, 90, 95, 100 mg/kg, or the range between any two of the above values, preferably 20-50 mg/kg; or,
the pathway modulator is Etamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Zamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fusaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Disulfiram or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fumaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg.

In the above method, the DBH inhibitor can be administered in combination with one or more selected from the group consisting of chemotherapeutic agent, targeted therapeutic agent, immunotherapy agent and anti-inflammatory agent.

In the above method, the medicament is preferably for use in one or more uses selected from the group consisting of: reducing the proportion of CD4+ T cells, increasing the proportion of regulatory T cells, reducing the secretion of pro-inflammatory factors of CD4+ T cells, inhibiting the activation of B cells and inhibiting the activation of NK cells.

In the above method, the pro-inflammatory factors secreted from CD4+ T cells are preferably one or more of IL-17A, IFN-γ and TNF-α;

In the above method, the pro-inflammatory factors secreted from CD8+ T cells are preferably IL-17A and/or TNF-α.

In the above method, the regulatory T cells are preferably CD25+FOXP3+ Treg cells.

In the above method, the B cells are preferably B220+ cells, more preferably CD69+B220+ B cells.

In the above method, the NK cells are preferably NK1.1+ cells, more preferably NK1.1+CD107a+ NK cells.

The eighth aspect of the present disclosure relates to a pharmaceutical composition comprising a pathway modulator and a pharmaceutically acceptable carrier; wherein, the pathway modulator is selected from the group consisting of DBH inhibitor, receptor agonist, receptor antagonist, and the combination thereof;
the pharmaceutical composition has one or more functions selected from the group consisting of: reducing the proportion of CD4+ T cells, increasing the proportion of regulatory T cells, increasing the proportion of CD8+ T cells, reducing the secretion of pro-inflammatory factors of CD4+ T cells, reducing the secretion of pro-inflammatory factors of CD8+ T cells, inhibiting the activation of B cells and inhibiting the activation of NK cells; reducing the content of lymphocytes, neutrophils and monocytes in the peripheral blood; reducing inflammatory cell infiltration and subdermal capillary hyperplasia in the dermis layer; ameliorating skin fibrosis; reducing the incidence of uveitis; ameliorating skin inflammation; improving stool form score, improving CW/CL, improving CW/BW, improving CW/CL/BW, inhibiting the increase of colon ulcer area, improving colon inflammatory cell infiltration score, improving tissue damage score; improving disease activity score, ameliorating hematochezia or occult blood.

In the above pharmaceutical composition, the pathway modulator is preferably a DBH inhibitor.

In the above pharmaceutical composition, the DBH inhibitor can be a DBH inhibitor disclosed in the prior art, and for example can be one or more of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof. The DBH inhibitor is preferably selected from the group consisting of Nepicastat, Etamicastat, Zamicastat, a pharmaceutically acceptable salt thereof, a prodrug thereof and the combination thereof. The DBH inhibitor is more preferably selected from the group consisting of Nepicastat, Etamicastat and Zamicastat.

In the above pharmaceutical composition, the DBH inhibitor is preferably selected from the group consisting of Nepicastat, a pharmaceutically acceptable salt thereof, a prodrug thereof and the combination thereof. The DBH inhibitor is more preferably Nepicastat.

In the above pharmaceutical composition, as examples:
the pathway modulator is Nepicastat or a pharmaceutically acceptable salt thereof, and the unit dose is 10-100 mg/kg, for example 10, 15, 20, 25, 30, 35, 40, 45, 50, 55, 60, 65, 70, 75, 80, 85, 90, 95, 100 mg/kg, or the range between any two of the above values, preferably 20-50 mg/kg; or,
the pathway modulator is Etamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Zamicastat or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fusaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Disulfiram or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg; or,
the pathway modulator is Fumaric acid or a pharmaceutically acceptable salt thereof, and the unit dose is 80-120 mg/kg, for example 80, 85, 90, 95, 100, 110, 120, or the range between any two of the above values, preferably 90-110 mg/kg, more preferably 100 mg/kg.

In the above pharmaceutical composition, the pharmaceutical composition can further comprise one or more substances selected from the group consisting of chemotherapeutic agent, targeted therapeutic agent, immunotherapy agent and anti-inflammatory agent.

In the above pharmaceutical composition, the medicament is preferably for use in one or more uses selected from the group consisting of: reducing the proportion of CD4+ T cells, increasing the proportion of regulatory T cells, reducing the secretion of pro-inflammatory factors of CD4+ T cells, inhibiting the activation of B cells and inhibiting the activation of NK cells.

In the above pharmaceutical composition, the pro-inflammatory factors secreted from CD4+ T cells are preferably one or more of IL-17A, IFN-γ and TNF-α;

In the above pharmaceutical composition, the pro-inflammatory factors secreted from CD8+ T cells are preferably IL-17A and/or TNF-α.

In the above pharmaceutical composition, the regulatory T cells are preferably CD25+FOXP3+ Treg cells.

In the above pharmaceutical composition, the B cells are preferably B220+ cells, more preferably CD69+B220+ B cells.

In the above pharmaceutical composition, the NK cells are preferably NK1.1+ cells, more preferably NK1.1+CD107a+ NK cells.

The beneficial effects of the present disclosure are: the inventors have found that DBH inhibitors can inhibit the development and progression of autoimmune diseases by immunoregulation (e.g., in particular, ameliorating weight loss, improving DAI score, and returning colon density to normal), thereby providing new choices for the treatment of autoimmune diseases.

### DESCRIPTION OF THE DRAWINGS

Figure 1A shows the results of body weight assessment in each group of mice, according to the experimental procedure described in Example 1.
Figure 1B shows the results of DAI score in each group of mice, according to the experimental procedure described in Example 1.
Figure 1C shows the results of colon density in each group of mice, according to the experimental procedure described in Example 1.
Figures 2A to 2E show the effects on CD4+ T cell subpopulation (Figure 2A), CD25+FOXP3+ Treg cell subpopulation (Figure 2B), IL-17A from CD4+ T cell subpopulation (Figure 2C), IFN-γ from CD4+ T cell subpopulation (Figure 2D), and TNF-α from CD4+ T cell subpopulation (Figure 2E) in mesenteric lymph nodes in each group of mice in Example 4.
Figures 3A to 3D show the effects on CD8+ T cell subpopulation (Figure 3A), IFN-γ from CD8+ T cell subpopulation (Figure 3B), IL-17A from CD8+ T cell subpopulation (Figure 3C), and TNF-α from CD8+ T cell subpopulation (Figure 3D) in mesenteric lymph nodes in each group of mice in Example 4.
Figures 4A to 4B show the effects on CD69+B220+ B cell subpopulation (Figure 4A) and NK1.1+CD107a+ NK cell subpopulation (Figure 4B) in mesenteric lymph nodes in each group of mice in Example 4. In the above figures, * refers to p<0.05, ** refers to p<0.01, **** refers to p<0.001, and **** refers to p<0.0001.
Figure 5 shows the body weight change in each group of Example 5; Two-way ANOVA: ^{#}p < 0.05, ^{###}p < 0.001 vs. solvent group.
Figure 6 shows the rate of body weight change in each group of Example 5.
Figure 7 shows the spleen weight in each group of Example 5; One-way ANOVA: ***p < 0.001 vs. normal group, ^{#}p < 0.05, ^{##}p < 0.01 vs. solvent group.
Figure 8 shows the spleen/body weight ratio in each group of Example 5; One-way ANOVA: ^{#}p < 0.05, ^{##}p < 0.01, ^{###}p < 0.001 vs. solvent group.
Figure 9 shows the red blood cell (RBC) count in peripheral blood in each group of Example 5; One-way ANOVA: ***p < 0.001 vs. normal group, ^{##}p < 0.01, ^{###}p < 0.001 vs. solvent group, ^{&&&}p < 0.001 vs. Imatinib group.
Figure 10 shows the hemoglobin (HGB) content in peripheral blood in each group of Example 5; One-way ANOVA: ***p < 0.001 vs. normal group, ^{##}p < 0.01, ^{###}p < 0.001 vs. solvent group, ^{&&&}p < 0.001 vs. Imatinib group.
Figure 11 shows the white blood cell (WBC) count in peripheral blood in each group of Example 5; One-way ANOVA: *p < 0.05, ***p < 0.001 vs. normal group, ^{##}p < 0.01 vs. solvent group.
Figure 12 shows the lymphocyte (LYMPH) count in peripheral blood in each group of Example 5; One-way ANOVA: #p < 0.05, ###p < 0.001 vs. solvent group.
Figure 13 shows the mononuclear cell (MONO) count in peripheral blood in each group of Example 5; One-way ANOVA: ##p < 0.01 vs. solvent group.
Figure 14 shows the neutrophil (NEUT) count in peripheral blood in each group of Example 5; One-way ANOVA: ***p < 0.001 vs. normal group.
Figure 15 shows the platelet content in peripheral blood in each group of Example 5; One-way ANOVA: ***p < 0.001 vs. normal group, ^{###}p < 0.001 vs. solvent group, ^{&&}p < 0.01, ^{&&&}p < 0.001 vs. Nintedanib group, ^{$$}p < 0.01 vs. Imatinib group.
Figure 16 shows the inflammatory cell infiltration score of dermal layer in each group of Example 5; One-way ANOVA: ^{#}p < 0.05 vs. solvent group, ^{$}p < 0.05 vs. Nepicastat-25 mg/kg, qd, po.
Figure 17 shows the capillary density score of the dermis layer in each group of Example 5; One-way ANOVA: ^{#}p < 0.05, ^{##}p < 0.01 vs. solvent group.
Figure 18 shows the dermal thickness -in each group of Example 5; One-way ANOVA: *p < 0.05, **p < 0.01, ***p < 0.001 vs. normal group.
Figure 19 shows the clinical score in each group of Example 6.
Figures 20A to 20D show the proportion of AQP4, GFAP, IBA1, CD45 positive cells in each group of Example 7.
Figure 21 shows the body weight of animals in each group of Example 8.
Figure 22 shows the AUC of erythema + scale + thickness total score in each group of Example 8; mean ± standard error, Two way ANOVA, p* < 0.05, p** < 0.01, p*** < 0.001, p**** < 0.0001 compared with the corresponding solvent group (note: the AUC of total score in normal group is 0, not shown in the Figure).
Figure 23 shows the spleen weight in each group of Example 8.
Figure 24 shows the body weight of animals in each group of Example 9.
Figure 25 shows the clinical score in each group of Example 9.
Figure 26 shows the AUC in each group of Example 9.
Figure 27 shows the incidence in each group of Example 9.
Figure 28 shows the body weight of animals in each group of Example 10.
Figure 29 shows the rate of body weight change of animals in each group of Example 10.
Figure 30 shows the stool score in each group of Example 10.
Figure 31 shows the AUC of stool score in each group of Example 10.
Figure 32 shows the body weight change in each group of Example 11.
Figure 33 shows the DAI in each group of Example 11.

### DETAILED DESCRIPTION OF THE INVENTION

Various publications and patent applications are cited in the background of the invention and throughout the specification, each of these references is incorporated herein by reference in its entirety. Unless otherwise defined, all technical and scientific terms used herein have the same meanings generally understood by those of ordinary skill in the art to which the present disclosure belongs.

### Terms

Herein, the term "receptor agonist" refers to a substance that can act on a receptor for a dopamine β-hydroxylase catalyzed substrate (i.e., dopamine) and exert the same mechanism and effect as elevated dopamine. The receptor agonist is preferably a dopamine receptor agonist.

Herein, the term "receptor antagonist" refers to a receptor that can act on receptor for a dopamine β-hydroxylase catalyzed product (i.e., norepinephrine and/or epinephrine) and exert the same mechanism and effect as reduced norepinephrine and/or epinephrine. The receptor antagonist is preferably a norepinephrine receptor antagonist and/or epinephrine receptor antagonist.

The term "autoimmune disease" is due to the attack on the body by its own immune system, characterized by disruption of the adaptive immune tolerance mechanism that identifies self/non-self and abnormal response of adaptive immune cells, leading to inflammatory damage to the body's own tissues.

The term "dopamine β-hydroxylase" is intended to encompass human derived dopamine β-hydroxylase and fragments, variants, precursors and functional domains thereof.

The term "DBH inhibitor" refers to any natural or artificial compound that can affect (which means reducing, lowering, inhibiting, blocking, suppressing, inactivating or preventing from activation) the structure, expression or activity of dopamine β-hydroxylase at the nucleic acid or protein level. DBH inhibitors include DBH inhibitors known in prior art as well as those available in the future. Included are DBH inhibitors disclosed in CN87103323A and WO9529165, as well as Nepicastat, Etamicastat, Zamicastat, Fusaric acid, Disulfiram, Cysteamine, Cysteamine derivatives, Pantethine and Pantethine derivatives.

The term "pharmaceutically acceptable carrier" refers to a pharmaceutically acceptable excipient.

The term "pharmaceutically acceptable salt" should be understood as referring to the following salts, which are pharmaceutically acceptable salts and which possess the expected pharmacological activity of the parent compound. Such salts include:
(1) Acid addition salts formed with inorganic acids, or acid addition salts formed with organic acids; wherein, the inorganic acids can be one or more of hydrochloric acid, hydrobromic acid, sulfuric acid, nitric acid and phosphoric acid; the organic acids can be formic acid, oxalic acid, succinic acid, acetic acid, benzenesulfonic acid, benzoic acid, camphorsulfonic acid, citric acid, ethanesulfonic acid, fumaric acid, glucoheptonic acid, gluconic acid, glutamic acid, glycollic acid, hydroxynaphthoic acid, 2-hydroxyethanesulfonic acid, lactic acid, maleic acid, malic acid, mandelic acid, methanesulfonic acid, muconic acid, 2-naphthalenesulfonic acid, propionic acid, salicylic acid, succinic acid, dibenzoyl-L-tartaric acid, tartaric acid, p-toluenesulfonic acid, trimethylacetic acid and trifluoroacetic acid; and
(2) Salts formed when acid protons present in the parent compound are substituted by metal ions, for example, alkali metal ions (e.g., Na⁺, K⁺ or Li⁺), alkaline earth metal ions (such as Ca²⁺ or Mg²⁺) or aluminum ions; or, when coordinated with organic or inorganic bases; wherein, the organic bases can be one or more organic bases of pyridines, imidazoles, pyrazines, indoles, purines, tertiary amines and anilines, preferably one or more of pyridine, methylpyridine, 4-dimethylaminopyridine, 2-methyl-5-ethylpyridine, triethylamine, N,N-diisopropylethanamine, N,N-dimethylaniline, diethanolamine, ethanolamine, N-methylglucosamine, triethanolamine and tromethamine; the inorganic bases can be one or more of aluminum hydroxide, calcium hydroxide, potassium hydroxide, sodium carbonate and sodium hydroxide.

The pharmaceutical composition of the present disclosure can be various conventional dosage forms, for example, tablet, aqueous suspension, oil suspension, dispersible powder, dispersible granule, emulsion, hard capsule, soft capsule, sterile aqueous solution for injection, sterile oil-in-water microemulsion for injection, or suppository. Each of the above dosage forms can be prepared by conventional preparation methods.

The excipient in the tablet of the present disclosure can be one or more of filler, binder, lubricant, flow aid and disintegrant. Wherein, the filler can be one or more of microcrystalline cellulose, starch, lactose monohydrate and dicalcium phosphate. The binder can be one or more of starch, gelatin, polyvinylpyrrolidone and gum arabic. The lubricant can be one or more of magnesium stearate, stearic acid and sodium lauryl sulfate. The flow aid can be one or two of colloidal silicon dioxide and talcum powder. The disintegrant can be one or more of crospovidone, sodium carboxymethyl starch, low-substituted hydroxypropylcellulose and croscarmellose sodium. The tablet can also contain a coating. The tablet can also be prepared into a sustained-release formulation, the sustained-release material in the sustained-release formulation can be one or two of hydroxypropyl methylcellulose and xanthan gum.

The excipient in the aqueous suspension of the present disclosure can be one or more of suspending agent, dispersant, preservative and flavoring agent. Wherein, the suspending agent can be one or more of sodium carboxymethylcellulose, methylcellulose, hydroxypropyl methylcellulose, sodium alginate, polyvinylpyrrolidone and gum arabic. The dispersant can be one or more of naturally occurring phospholipid (e.g., lecithin), condensation product of alkylene oxide with fatty acid (e.g., polyoxyethylene stearate), condensation product of ethylene oxide with long chain fatty alcohol (e.g., heptadecaethyleneoxy cetanol), condensation product of ethylene oxide with partial ester derived from fatty acid and hexitol (e.g., polyethylene oxide sorbitol monooleate), condensation product of ethylene oxide with partial ester derived from fatty acid and hexitol anhydride (e.g., polyethylene oxide sorbitan monooleate). The preservative can be ethylparaben and/or n-propylparaben. The flavoring agent can be one or more of sucrose, saccharin and aspartame.

The excipient in the oil suspension of the present disclosure can be one or more of suspending agent, thickener, flavoring agent and antioxidant. The suspending agent can be vegetable oil and/or mineral oil, the vegetable oil can be one or more of peanut oil, olive oil, sesame oil and coconut oil, and the mineral oil can be liquid paraffin. The thickener can be one or more of beeswax, hard paraffin and cetyl alcohol. The flavoring agent can be one or more of sucrose, saccharin and aspartame. The antioxidant can be one or more of butylated hydroxyanisole, α-tocopherol and ascorbic acid.

The excipient in the dispersible powder and dispersible granule of the present disclosure can be one or more of suspending agent, dispersant, preservative, flavoring agent and antioxidant. The specific selection of the above components is the same as the excipient in the aqueous suspension.

The excipient in the emulsion of the present disclosure can be one or more of suspending agent, emulsifier, flavoring agent, preservative and antioxidant. The suspending agent can be vegetable oil and/or mineral oil, the vegetable oil can be olive oil and/or peanut oil, and the mineral oil can be liquid paraffin. The emulsifier can be one or more of naturally occurring phospholipid (e.g., soy lecithin), ester or partial ester derived from fatty acids and hexitol anhydrides (e.g., sorbitan monooleate), and condensation product of the partial ester and ethylene oxide (e.g., polyethylene oxide sorbitol monooleate). The flavoring agent can be one or more of glycerol, propylene glycol, sorbitol and sucrose. The preservative can be ethylparaben and/or n-propylparaben. The antioxidant can be one or more of butylated hydroxyanisole, α-tocopherol and ascorbic acid.

The excipient in the hard capsule of the present disclosure can be a conventional inert solid thinner, for example, it can be one or more of calcium carbonate, calcium phosphate and kaolin.

The excipient in the soft capsule of the present disclosure can be a conventional water-soluble carrier and/or conventional oil solvent, for example, it can be one or more of polyethylene glycol, peanut oil, liquid paraffin and olive oil.

The excipient in the sterile aqueous solution for injection of the present disclosure can be a pharmaceutically acceptable solvent, for example, water, Ringer's solution or isotonic sodium chloride solution.

The excipient in the sterile oil-in-water microemulsion for injection of the present disclosure can be an oil-phase excipient and aqueous-phase excipient, the oil-phase excipient can be a mixture of soybean oil and lecithin, and the aqueous-phase excipient can be a mixture of water and glycerol.

The excipient in the suppository of the present disclosure can be one or more of cocoa butter, glycerol, gelatin, hydrogenated vegetable oil, polyethylene glycol and fatty acid ester of polyethylene glycol.

The term "subject" refers to an animal, preferably a mammal. According to particular embodiments, the subject is a mammal, including, for example, camel, donkey, zebra, cattle, pig, horse, goat, sheep, cat, dog, rat, rabbit, guinea pig, mouse, primate (e.g., human). In particular embodiments, the subject is a human. In particular embodiments, the subject is a human who is susceptible to, suspected of having, or has suffered from an autoimmune disease.

The term "treatment" refers to eliminating the disease, preventing disease progression, slowing disease progression, reducing the duration of one or more symptoms associated with the disease, improving or reversing at least one measurable parameter associated with the disease, or increasing the survival of subjects with the disease.

The term "effective amount" refers to an amount of the active ingredient of the drug that elicits the desired effect in a subject. In particular embodiments, those skilled in the art can determine the selection of an effective amount based on consideration of a variety of factors (e.g., through clinical trials), the factors include the disease to be treated, the symptoms involved, the route of administration, the severity of the disease, the patient's body weight, the patient's immune status, and other factors known to those skilled in the art. The effective amount of in a particular embodiment can be obtained from the dose-response curve derived from an animal model testing system, and allows to be determined according to the opinion of a physician and the situation of each patient. Wherein, the correlation between animal and human doses is described in Freireich *et al.*, 1966, Cancer Chemother Rep 50:219, and the body surface area of human can be approximately determined by the height and body weight of the patient. The effective amount of the drug compounds of the present disclosure can be 0.5 mg/kg to 500 mg/kg, preferably 1 mg/kg to 200 mg/kg, more preferably 10 mg/kg to 100 mg/kg.

Herein, the same pharmaceutical active ingredient (which refers to a single drug compound) or different pharmaceutical active ingredients (which refers to two or more drug compounds) can be administered at one time, or can be divided into many smaller unit doses, administered at a certain time interval. It should be understood that the exact dose, duration, and interval of treatment is a function of the disease being treated, and can be determined by inference using animal or clinical trial data. "Administer", "administered", "administering" or "administration" can include a single administration, or two or more administrations at an appropriate time interval. Wherein, the time interval between two administrations can be 30 minutes, 40 minutes, 50 minutes, 60 minutes, 2 hours, 3 hours, 4 hours, 5 hours, 6 hours, 7 hours, 8 hours, 9 hours, 10 hours, 12 hours, 14 hours, 16 hours, 18 hours, 20 hours, 22 hours, 24 hours, one and a half days, 2 days, 3 days, 4 days, 5 days, 6 days, 7 days, 8 days, 9 days, 10 days, 1 week, 2 weeks, 3 weeks, 4 weeks, 5 weeks, 6 weeks, 1 month, 2 months, 3 months , 4 months, 5 months, 6 months, 7 months, 8 months, 9 months, 10 months, 11 months or 12 months.

Each pharmaceutical active ingredient (each drug compound) mentioned herein can be used as the only active compound, or can be administered in combination with other active compounds (which refers to compounds other than the drug compounds described herein), as long as they do not produce other adverse effects, such as allergic responses. "Administered in combination with" or "combined administration" includes simultaneous or sequential administration of each active compound.

The term "administered in combination with" or "combined administration" refers to a method of providing two or more active compounds simultaneously or sequentially to a subject for therapeutic purposes. When "administered in combination with" or "combined administration" is involved, the time interval between each administration is sufficient to achieve synergy between each active compound administered. Two or more active compounds are in the same or different containers.

Abbreviations: QD: once a day; BID: twice a day; PO: oral; IP: intraperitoneal injection; IM: intramuscular injection.

### Example 1: Inhibition of DBH inhibitors on dextran sulfate sodium (DSS)-induced colitis

### 1. Materials and instruments:

**Table 1. Reagents**

| Reagent | Supplier | Catalog No. |
|---|---|---|
| Dulbecco's Phosphate Buffered Saline (1× DPBS) | Coming Incorporated | 21-031-CVR |
| Dextran sulfate sodium (DSS) | MP Biomedicals | SR01606 |
| Cyclosporin A (CsA) | Novartis | / |
| Normal saline | Ke Lun Pharmaceutical Co., Ltd | / |
| PEG400 | Sigma Aldrich | 25322-68-3 |
| 30% Solutol HS15 | Sigma Aldrich | 70142-34-6 |
| Nepicastat | Selleckchem | S2695 |
| Etamicastat | Nanjing PharmaBlock | 677773-32-9 |
| Zamicastat | MCE | 1080028-80-3 |

**Table 2. Instruments**

| Instrument | Supplier | Type |
|---|---|---|
| Electronic balance | Changzhou Tianzhiping | YH-2000 |
| Electronic analytical balance | Mettle Toledo | 585310 |

### 2. Preparation and storage of drug solution:

The composition of the solvent was as follows: 20 v% PEG400 + 10 v% (30% Solutol HS15) + 70 v% normal saline.

Substance to be tested - Nepicastat solution: 27 mg of Nepicastat was completely dissolved in 9 mL of solvent (this concentration corresponded to Test group 1#), prepared every 3 days, and stored at 4°C to maintain the effect.

Substance to be tested - Etamicastat solution: 9 mg, 27 mg, 90 mg of Etamicastat were completely dissolved in 9 mL of solvent respectively (the three concentrations corresponded to Test group 2#, Test group 3#, and Test group 4#, respectively), prepared every 3 days, and stored at 4°C to maintain the effect.

Substance to be tested - Zamicastat solution: 9 mg, 27 mg, 90 mg of Zamicastat were completely dissolved in 9 mL of solvent respectively (the three concentrations corresponded to Test group 5#, Test group 6#, and Test group 7#, respectively), prepared every 3 days, and stored at 4°C to maintain the effect.

Positive drug control - Cyclosporin A solution: 200 mg of Cyclosporin A was completely dissolved in 10 mL of normal saline and prepared into a 20 mg/mL solution (corresponding to the positive drug control group), prepared every 3 days, and stored at 4°C to maintain the effect.

### 3. Treatment of mice in each group:

8-10 week old female C57BL/6J mice purchased from Charles River Laboratories were used. The mice were housed in a room at constant temperature (20±2°C) (5 mice per cage) with a 12-hour light-dark cycle. All protocols were approved by the Institutional Animal Care and Use Committee of WuXi AppTec. These mice were adapted to the housing conditions at the animal experiment center of WuXi AppTec for at least three days prior to the experiment.

### (1) Solvent group, positive drug control group and test groups

In order to establish a model of dextran sulfate sodium-induced colitis, mice were given free access to 3% DSS aqueous solution (3 g DSS was added to 100 mL of water to prepare this aqueous solution, wherein the molecular weight of DSS was 36000-50000) as drinking water for 7 consecutive days to induce colitis (which belongs to autoimmune colitis), and divided into 9 groups (see the solvent group, positive drug control group and Test group 1# to Test group 7# in Table 3), wherein the 3% DSS aqueous solution was freshly prepared every day. At the experimental endpoint (see hereinafter), mice were euthanized and endpoint samples were obtained.

### (2) Normal group

Mice in the normal group were not given 3% DSS aqueous solution to induce colitis and were free to drink blank water. At the experimental endpoint (see hereinafter), mice were euthanized and endpoint samples were obtained.

**Table 3. Groups and dosing regimen**

| Group | Number of mice | Experimental agent | Dose (mg/kg) | Dosing regimen | |
|---|---|---|---|---|---|
| | | | | Route | Frequency |
| Normal group | 10 | Water | / | - | - |
| Solvent group | 10 | Solvent | / | PO | QD × 8 days |
| Positive drug control group | 10 | Cyclosporine A and solvent | 50 | IM | QD × 8 days |
| Test group 1# | 10 | Nepicastat and solvent | 30 | PO | QD × 8 days |
| Test group 2# | 10 | Etamicastat and solvent | 10 | PO | QD × 8 days |
| Test group 3# | 10 | Etamicastat and solvent | 30 | PO | QD × 8 days |
| Test group 4# | 10 | Etamicastat and solvent | 100 | PO | QD × 8 days |
| Test group 5# | 10 | Zamicastat and solvent | 10 | PO | QD × 8 days |
| Test group 6# | 10 | Zamicastat and solvent | 30 | PO | QD × 8 days |
| Test group 7# | 10 | Zamicastat and solvent | 100 | PO | QD × 8 days |
| In the solvent group, each mouse was given the solvent at 10 mL/kg body weight every day. | | | | | |

### 4. Body weight assessment and disease activity index (DAI) score:

The animal body weight and DAI score were assessed under double-blind conditions, that is, the researchers collected the body weight data of mice and observed and scored the stool characteristics and blood stools every day, without knowing the group and dosing regimen. The DAI score was the sum of the weight loss score, stool score and bleeding score. The scoring criteria were as shown in Table 4.

**Table 4. DAI scoring system**

| Scoring system | | | |
|---|---|---|---|
| Score | Weight loss | Hardness of stool | Blood stool |
| 0 | No weight loss | Normal | Occult blood negative |
| 1 | 1-5% | Soft but shaped | Occult blood weak positive |
| 2 | 5-10% | Very soft | Occult blood positive |
| 3 | 10-20% | Diarrhea | Bleeding |
| 4 | >20% | Severe diarrhea | Severe bleeding |

### 5. Statistical analysis:

The data were analyzed by analysis of variance, specifically by using the following process: using Graph Pad Prism 6.0 software to carry out post-hoc Dunnett's multiple comparison test. The other groups (referring to the normal group, positive drug control group and Test groups 1# to 7#) were compared with the solvent group to analyze whether the other groups had significance compared with the solvent group. If the p-value < 0.05, then the groups were statistically different and had significance. Data were expressed as mean ± S.E.M.

### Example 2: Effects of DBH inhibitors on immune cell activation and cytokines

### 1. Materials:

**Table 5. Antibody reagents for flow cytometry**

| No. | Target of antibody | Stain | Supplier | Catalog No. | Full name |
|---|---|---|---|---|---|
| 1 | CD45 | APC-Cy7 | BD bioscience | 557659 | APC-Cy^{™}7 rat-anti-mouse CD45 |
| 2 | CD3 | BV510 | BD bioscience | 563024 | BD Horizon^{™} BV510 hamster-anti-mouse CD3e |
| 3 | CD4 | BUV395 | BD bioscience | 563790 | BD Horizon^{™} BUV395 rat-anti-mouse CD4 |
| 4 | CD8 | AF700 | BD bioscience | 557959 | Alexa Fluor^{®} 700 rat-anti-mouse CD8a |
| 5 | CD25 | BV421 | BD bioscience | 562606 | BV421 rat-anti-mouse CD25 |
| 6 | IFN-γ | BV650 | BD bioscience | 563854 | BD Horizon^{™} BV650 rat-anti-mouse IFN-γ |
| 7 | IL-17A | BV786 | BD bioscience | 564171 | BD Horizon^{™} BV786 rat-anti-mouse IL-17A |
| 8 | Foxp3 | FITC | eBioscience | 11-5773-82 | FOXP3 monoclonal antibody (FJK-16s), FITC, eBioscience^{™} |
| 9 | TNF-α | BB700 | BD bioscience | 566510 | BD Horizon^{™} BB700 rat-anti-mouse TNF |
| 10 | Viable/dead cells | FVS620 | BD bioscience | 564996 | BD Horizon^{™} Fixable Cell Viability Stain 620 |
| 11 | CD45RO /B220 | FITC | BD bioscience | 553087 | BD Pharmingen^{™} FITC rat-anti-mouse CD45R/B220 |
| 12 | CD3 | PE | BD bioscience | 553064 | BD Phartmingen^{™} PE hamster-anti-mouse CD3e |
| 13 | NK1.1 | BV421 | BD bioscience | 562921 | BD Horizon^{™} BV421 mouse-anti-rat NK-1.1 |
| 14 | CD69 | BV786 | BD bioscience | 564683 | BD Horizon^{™} BV786 hamster-anti-mouse CD69 |
| 15 | CD107a | PECY7 | BD bioscience | 560647 | BD Pharmingen^{™} PE-Cy^{™}7 rat-anti-mouse CD107a |
| Each of the above reagents was used at a volume of 2 µL per use. | | | | | |

**Table 6. Other reagents**

| Reagent/material | Supplier | Catalog No. |
|---|---|---|
| 1640 Medium | Invitrogen | 22400-089 |
| Fetal bovine serum | Hyclone | SV30087.03 |
| FC Block | BD bioscience | 553141 |
| Intracellular Fixation & Permeabilization Buffer (which could be used as cell fixation buffer, cell permeabilization buffer and cell permeabilization washing buffer) | ebioscience | 85-88-8824-00 |
| Cell Activation Cocktail | Biolegend | 423304 |
| Cell counting slides | Invitrogen | C10228 |
| Antibody stain buffer for flow cytometry | BD bioscience | 563794 |

### 2. Methods:

The experimental endpoint was 24 hours after administration on Day 8 for the positive drug control group and the Test group 1# to Test group 7# of Example 1; the experimental endpoint of the normal group and the solvent group was the same as that of the positive drug control group and Test group 1# to Test group 7#.

At the experimental endpoint, mesenteric lymphatic nodes were collected and treated as follows:
1) The mesenteric lymph nodes were gently grinded and filtered with a 70 µM strainer (BD bioscience, Catalog No. 352350) to obtain a cell suspension, and the cells were counted.
2) The cells were suspended in a suspension solution (the suspension solution was prepared by the following process: diluting the cell activation mixture with a mixture of 90 v% 1640 medium and 10 v% fetal bovine serum to a concentration of 1×), the cell density was adjusted to 2.5×10⁷/mL, and the cells were incubated at 37°C for 5 hours.
3) The cells were washed with Dulbecco's Phosphate Buffered Saline, and stained with viable/dead stain (stain No. 12 in Table 5) at room temperature for 15 min to distinguish between viable cells and dead cells; the cells were washed with staining buffer (prepared by mixing fetal bovine serum and Dulbecco's Phosphate Buffered Saline at a volume ratio of 2:98), Fc block working solution (prepared by mixing Fc block and staining buffer at a volume ratio of 1:200) was added, and the cells were incubated at room temperature for 15 minutes.
4) The cells were surface labeled with 100 µL of antibody staining solution for flow cytometry without washing and incubated at 4°C for 30 minutes.
5) The cells were washed once with staining buffer, 100 µL of cell fixative solution was added, and the cells were incubated at 4°C overnight.
6) 200 µL of cell permeabilization solution was added and incubated at 4°C for 30 minutes.
7) After washing with 250 µL of cell permeabilization solution, the cells were suspended with 100 µL of staining buffer and fluorescence was detected with a BD LSRFortessa instrument.
8) The cell subpopulations analyzed by flow cytometry included CD4+ T cell subpopulation, IL-17 A +CD4+ T cell subpopulation, IFN-y+CD4+ T cell subpopulation, TNF-α+CD4+ T cell subpopulation, CD25+FOXP3+ Treg cell subpopulation; CD8+ T cell subpopulation, IL-17A+CD8+ T cell subpopulation, IFN-y+CD8+ T cell subpopulation, TNF-α+CD8+ T cell subpopulation; CD69+B220+ B cell subpopulation and NK1.1+CD107a+ NK cell subpopulation.

### Example 3: DBH inhibitors ameliorated DSS-induced colon injury

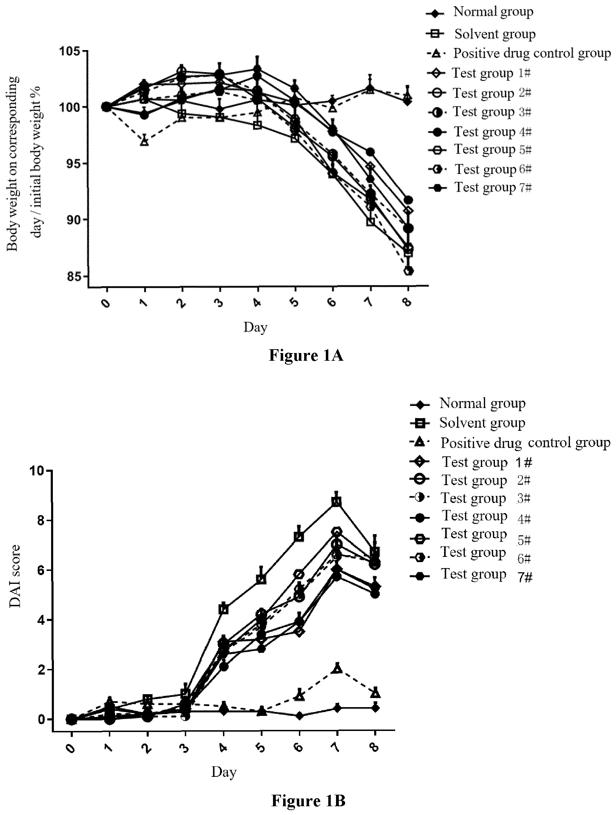

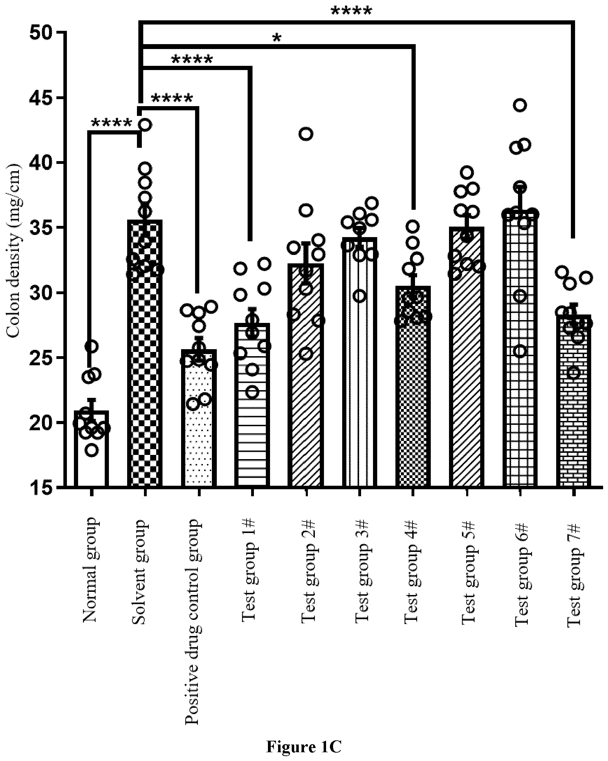

After performing the experiment according to the experimental procedure described in Example 1, the body weight assessment results in each group of mice were shown in Table 7 and Figure 1A; the DAI score results in each group of mice were shown in Table 8 and Figure 1B; the colon density results in each group of mice were shown in Table 9 and Figure 1C.

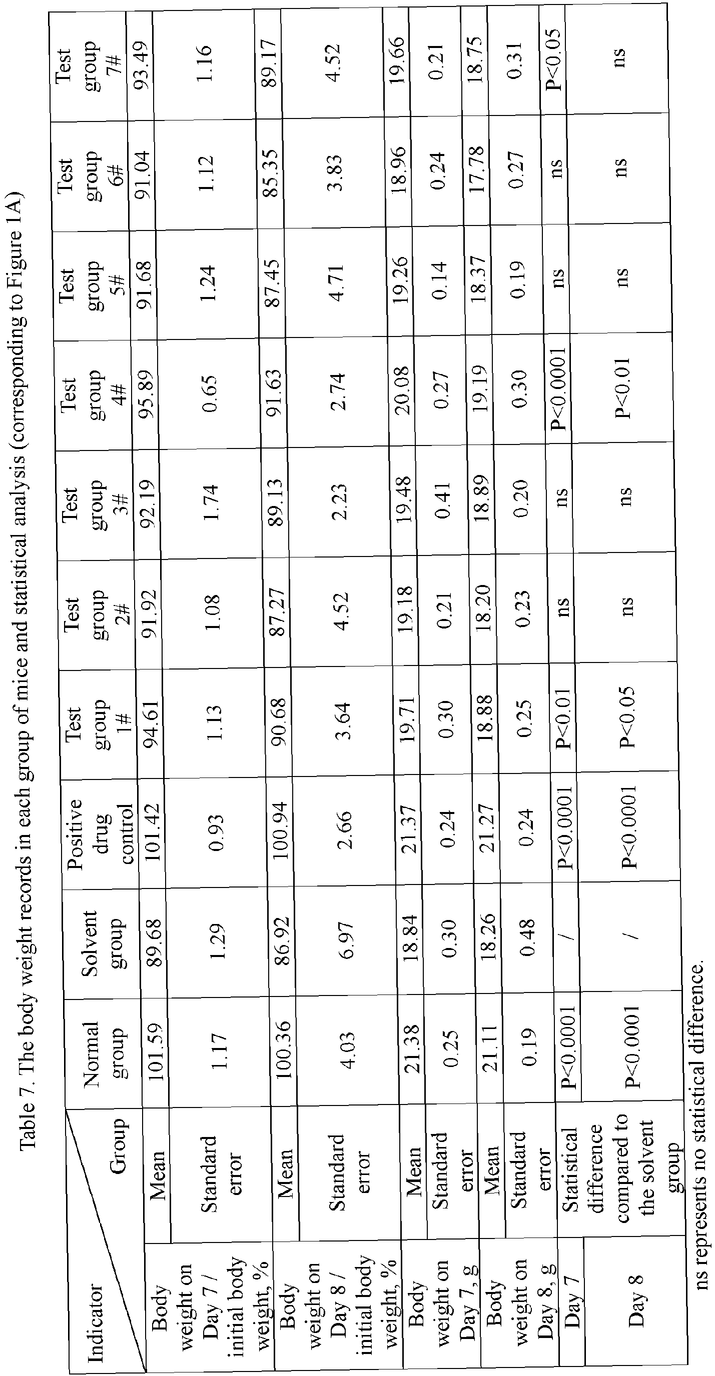

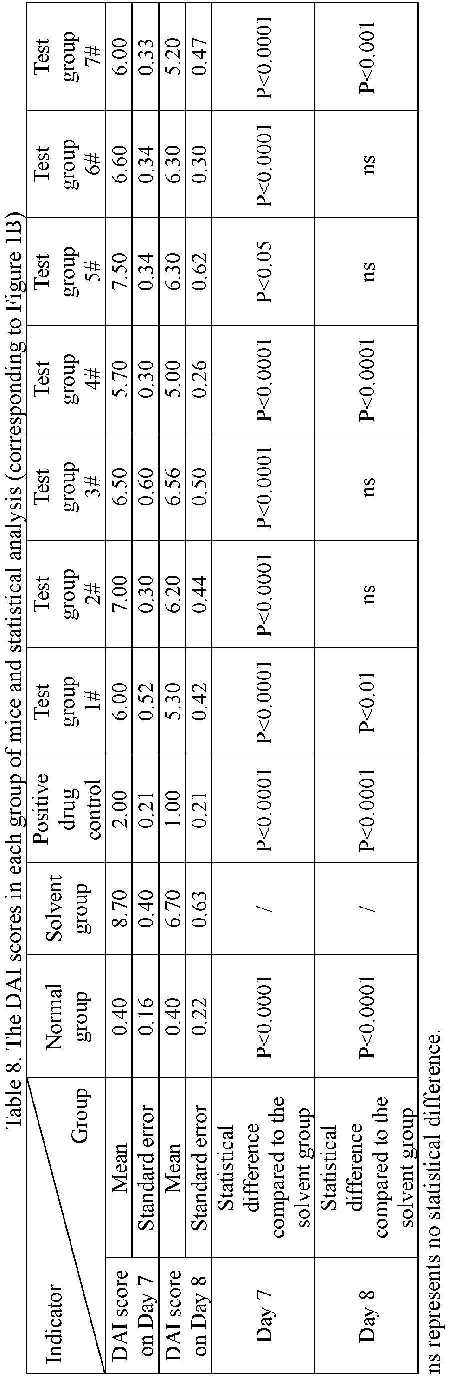

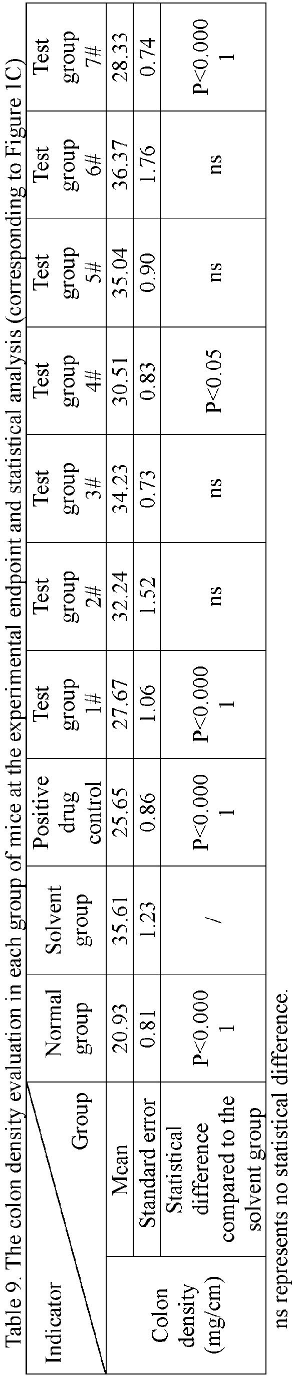

Mice in Test group 1#, Test group 4# and Test group 7# all had significantly less weight loss on Day 7 of treatment compared with the solvent group (Figure 1A, Table 7).

Mice in Test group 1#, Test group 2#, Test group 3#, Test group 4#, Test group 5#, Test group 6# and Test group 7# all had significantly lower DAI scores on Day 7 of treatment compared with the solvent group (Figure 1B, Table 8).

DSS administered to mice usually leads to a shortened colon and an increased colon density, which are two indicators that can reflect the severity of DSS-induced colitis. The colon density of Test group 1#, Test group 4# and Test group 7# was significantly reduced compared with the solvent group (Figure 1C, Table 9). These data suggested that Nepicastat, Etamicastat and Zamicastat ameliorated the severity of DSS-induced colitis and reduced disease activity.

In summary, DSS-induced colitis in Test group 1#, Test group 4# and Test group 7# was milder than that in the solvent group, suggesting that Nepicastat, Etamicastat and Zamicastat have potential therapeutic effects on colitis.

### Example 4: Effects of Nepicastat and Zamicastat on immune cells and cytokines

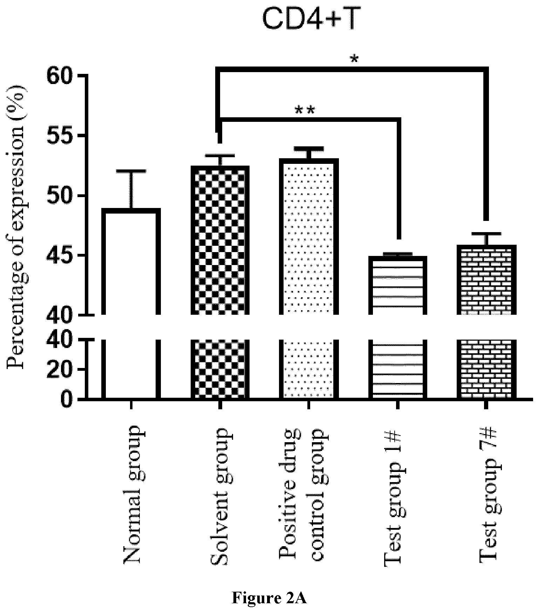

According to the method of Example 2, the effects of Nepicastat and Zamicastat on immune cells and cytokines were analyzed, and the results obtained were shown in Figures 2A to 2E, Figures 3A to 3D, and Figures 4A to 4B.

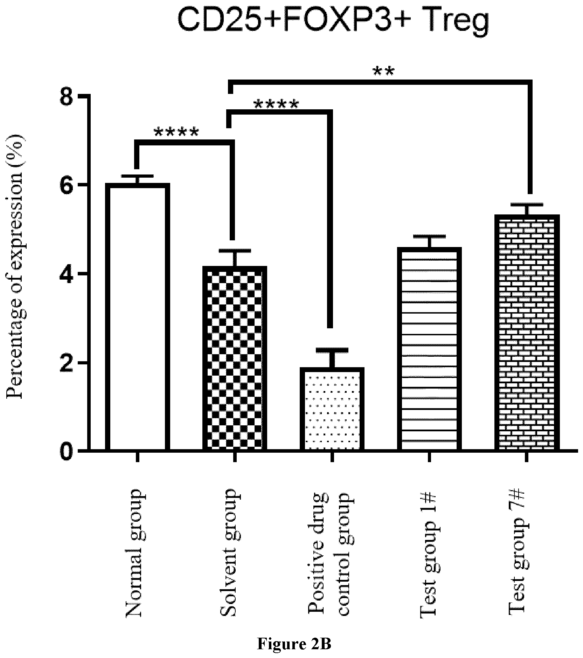

First, the changes of CD4+ T cell subpopulation from CD3+CD45+ cell subpopulation were analyzed, and the changes of CD25+FOXP3+ Treg cell subpopulation from CD4+ cell subpopulation were also analyzed. The results showed that Test group 1# and Test group 7# could reduce the proportion of CD4+ T cell subpopulation (Figure 2A) and increase the proportion of CD25+FOXP3+ Treg cell subpopulation (Figure 2B) compared with the solvent group. The above results suggested that DBH inhibitors could significantly inhibit inflammation and immunity.

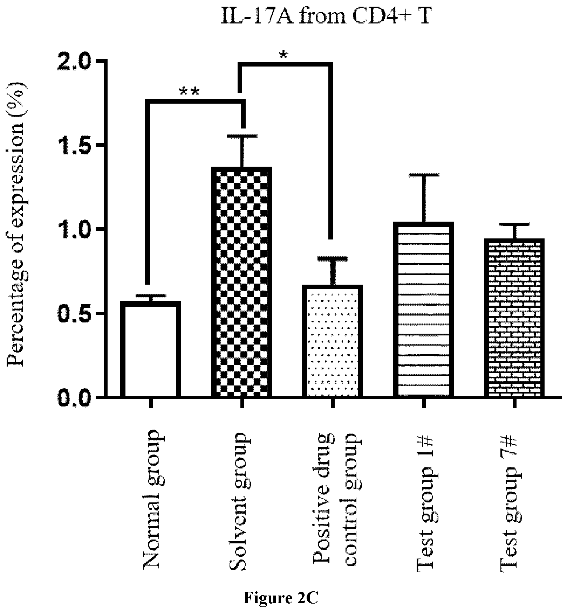

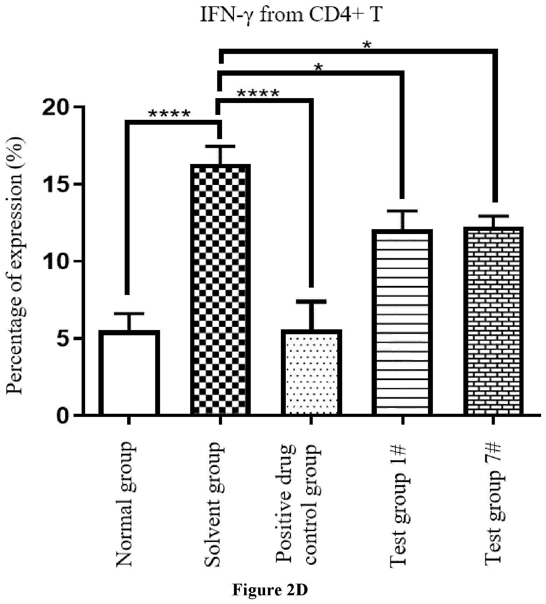

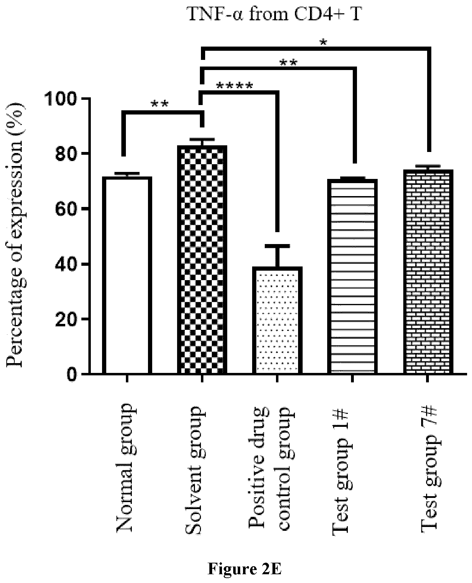

Then, the cytokines secreted by the CD4+ T cell subpopulation were further analyzed. The results showed that both the Test group 1# and Test group 7# could reduce the secretion of pro-inflammatory factors IL-17A (Figure 2C), IFN-γ (Figure 2D) and TNF-α (Figure 2E) compared with the solvent group, further indicating that DBH inhibitors inhibited inflammation and immunity by reducing the secretion of pro-inflammatory factors by CD4+ T cell subpopulation.

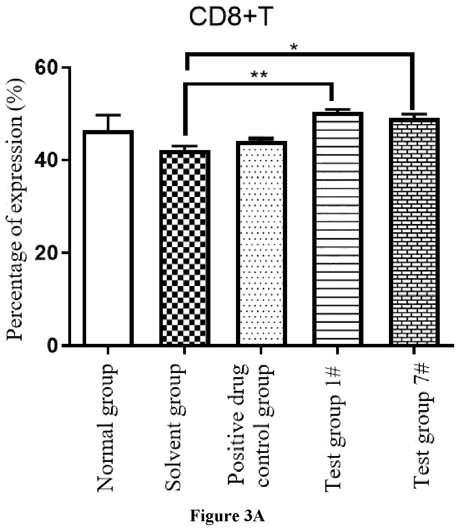

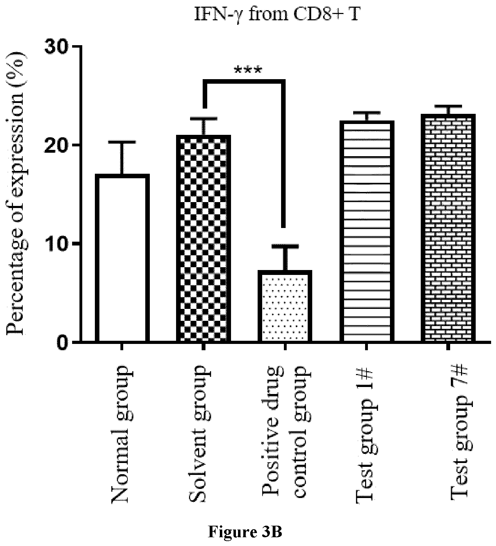

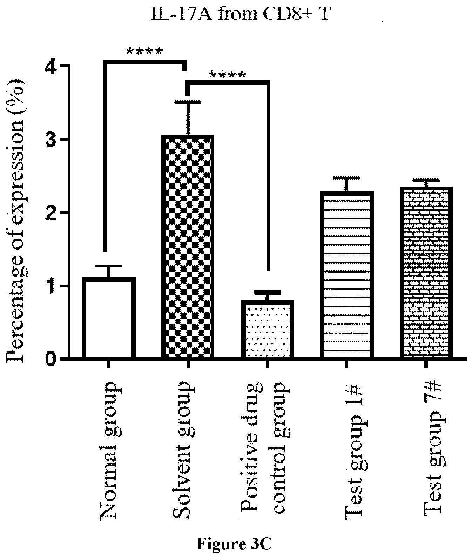

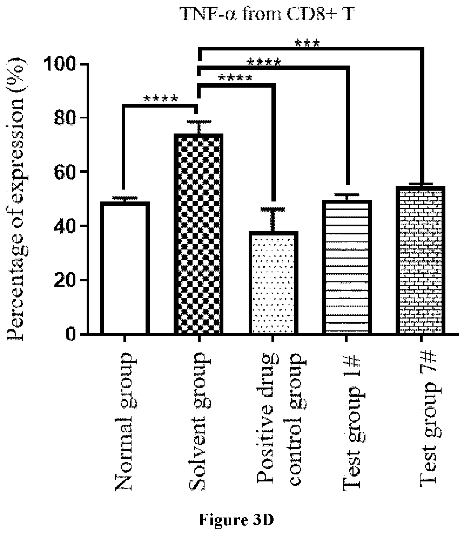

In addition, CD8+ T cell subpopulation from the CD3+CD45+ cell subpopulation and their secreted cytokines were similarly analyzed. The results showed that DBH inhibitors increased the proportion of CD8+ T cell subpopulation compared with the solvent group (Figure 3A), had no significant effect on the secretion of IFN-γ (Figure 3B), but inhibited the secretion of IL-17A (Figure 3C) and TNF-α (Figure 3D). The above results suggested that DBH inhibitors inhibited inflammation and immunity by reducing the secretion of IL-17A and TNF-α by CD8+ T cell subpopulation.

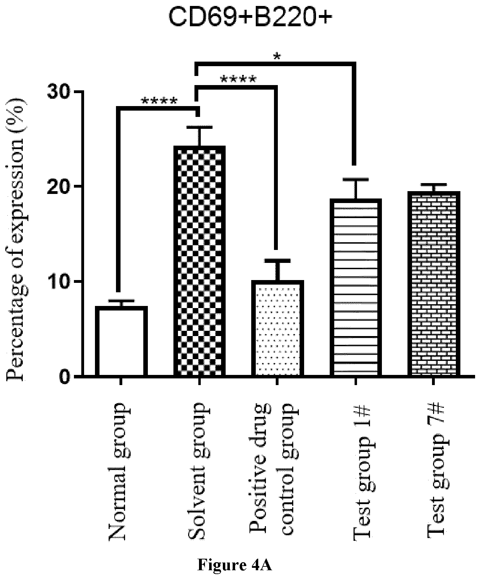

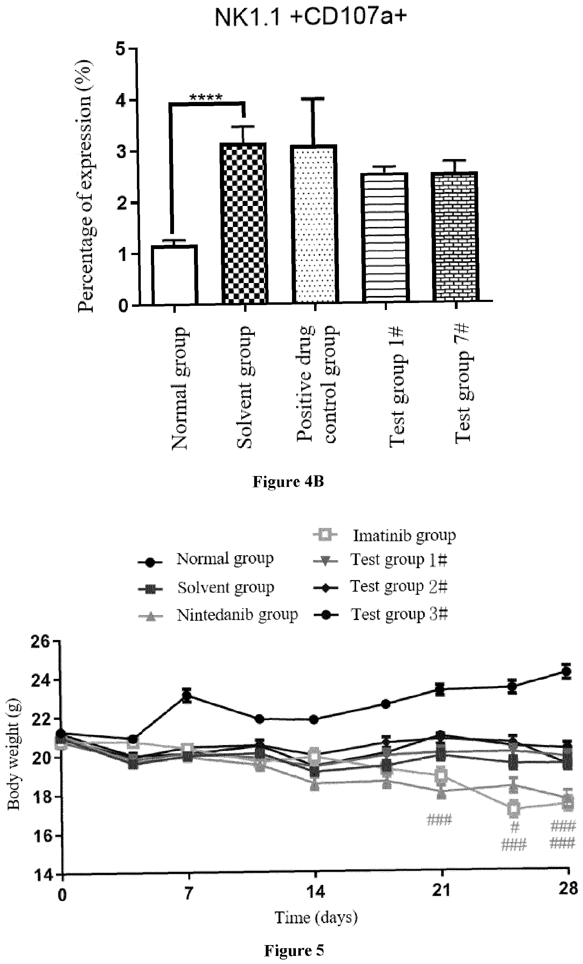

Finally, the activation of B cells (referring to the CD69+B220+ B cell subpopulation from the CD3- cell subpopulation) and NK cells (referring to the NK1.1+CD107a+ NK cell subpopulation from the CD3-B220- cell subpopulation) were evaluated. The results showed that all DBH inhibitors could inhibit the activation of B cells (Figure 4A) and NK cells (Figure 4B) compared with the solvent group. The above results suggested that DBH inhibitors could reduce the risk of B cells producing autoantibodies and NK cell-mediated non-specific killing of mucosal tissues, thereby alleviating inflammation and suppressing immunity.

### Example 5: Inhibition of DBH inhibitors on bleomycin-induced skin fibrosis in male C57 mice

### 1. Materials and instruments

**Table 10. Reagents**

| Reagent | Supplier | Catalog No. |
|---|---|---|
| Normal saline | Zhejiang Dubang Pharmaceuticals Co., Ltd. | 2009240101 |
| Solutol HS15 | Sigma | BCCF2234 |
| PEG400 | Sinopharm Chemical Reagent Co., Ltd. | 20181205 |
| MC | Sigma | SLBT4343 |
| Tween 80 | Sigma | WXBD2914v |
| Isoflurane | RWD | 20120101 |
| Bleomycin hydrochloride | Nippon Kayaku Co., Ltd. | 600700 |

**Table 11. Instruments**

| Name | Type and Manufacturer |
|---|---|
| Anesthesia respirator | AMS (Gene&I) Beijing GENE&I Technology Co., Ltd. |
| Automated Hematology Analyzer | Sysmex XS-800i |
| Tissue processor | LEICA HistoCore Pearl |
| Embedding instrument | Leica Histocore Arcadia C&H |
| Sectioning instrument | LEICA, RM2235 |
| Automatic stainer | LEICA, ST5020 |
| Slide scanner | HAMAMATSU Nano Zoomer S210 |
| CNC ultrasonic cleaner | KQ-100DE, Kunshan Ultrasonic Instruments Co., Ltd. |

### 2. Preparation and storage of drug solution

Composition of the solvent: 20% PEG400 + 10% (30% Solutol HS15) + 70% normal saline. 100 mL of PEG400 and 50 mL of 30% (V/V) Solutol were measured and added to 350 mL of normal saline. The mixture was placed on a magnetic stirrer and stirred until thoroughly mixed, and stored at 4°C for later use.

Substance to be tested - Nepicastat solution: 40 mg of the substance to be tested was weighed and put into a brown sample vial, and 0.8 mL of PEG400 was added. The vial was vortexed on a vortex, ultrasonicated for 15 minutes, and heated in a water bath at 40°C for 15 minutes, resulting in a suspension. Then 0.4 mL of 30% Solutol HS15 was added and the vial was vortexed on the vortex until thoroughly mixed. Then 2.8 mL of normal saline was added and the vial was vortexed on the vortex until thoroughly mixed to form a solution, in which the compound concentration was 10 mg/mL. The solution was prepared every three days and stored at 4°C for later use.

Reference substance - Nintedanib solution: 25 mg of Nintedanib (BIBF1120) was accurately weighed and added to 5 mL of solvent (0.5% MC + 0.2% Tween80). The mixture was thoroughly mixed until the solution was clear and transparent, in which the drug concentration was 5 mg/mL (the dose of administration was 10 mL/kg). The solution was prepared every seven days.

Reference substance - Imatinib solution: 15 mg of Imatinib mesylate powder was accurately weighed and put into a sample vial, and 3 mL of normal saline was added. The vial was vortexed on a vortex until the substance was completely dissolved, and the drug concentration was 5 mg/mL. The solution was freshly prepared before use and used within half an hour.

### 3. Experimental methods

### 3.1 Animal housing

70 male C57BL/6 mice (body weight 20 to 22 g) were housed in the SPF barrier system of KCI Biotechnology (Suzhou) Co., Ltd. under animal use certificate No. SYXK (Su) 2017-0041, in accordance with the international standard for temperature, humidity, light control systems.

The animal operation protocol of this experiment was approved and confirmed by the IACUC Committee. Operation and management strictly followed the SOP of KCI Biotechnology (Suzhou) Co., Ltd.

### 3.2 Model establishment

Isoflurane (2.0 to 2.5%) was used to anesthetize the animals. The fur of the back was removed, and a 1 cm² area was selected for intradermal injection of bleomycin (0.3 mg/kg, 100 µL) every two days to establish a skin fibrosis model.

### 3.3 Experimental grouping

According to their body weight, the animals were divided into 7 groups of 10 animals in each group, namely the normal group, solvent group, Nintedanib group (positive control drug), Imatinib (positive control drug), Test group 1#, Test group 2# and Test group 3#, as shown in Table 12.

**Table 12. Experimental grouping and dosing regimen**

| Group | Number of animals | Model establish -ment | Drug | Dose of administration | Route of administration | Volume of administration |
|---|---|---|---|---|---|---|
| Normal group | 10 | No | Solvent^{a} | NA | PO, QD | 10 mL/kg |
| Solvent group | 10 | Yes | Solvent^{a} | NA | PO, QD | 10 mL/kg |
| Nintedanib group | 10 | Yes | Nintedanib | 50 mg/kg | PO, BID | 10 mL/kg |
| Imatinib group | 8 | Yes | Imatinib | 50 mg/kg | IP, QD | 10 mL/kg |
| Test group 1# | 10 | Yes | Nepicastat | 25 mg/kg | PO, QD | 10 mL/kg |
| Test group 2# | 10 | Yes | Nepicastat | 50 mg/kg | PO, QD | 10 mL/kg |
| Test group 3# | 10 | Yes | Nepicastat | 100 mg/kg | PO, QD | 10 mL/kg |

| | | | | | | |
|---|---|---|---|---|---|---|
| Note: a: 20% PEG400 + 10% (30% Solutol HS15) + 70% normal saline. | | | | | | |

### 3.4 Experimental dosing

Dosing started on the day of model establishment. Each animal was weighed for the animal body weight before dosing to calculate the volume of administration, wherein the period of administration was 28 days. Route of administration: intraperitoneal injection for Imatinib group and gavage for other groups. Frequency of administration: twice a day for Nintedanib group and once a day for other groups.

### 3.5 Physiological observation of experimental animals

The change in animal body weight was recorded twice a week from the date of model establishment. The clinical manifestations of animals, such as shortness and difficulty of breath, abdominal suction, decreased activity and malaise, were closely observed.

### 3.6 Local skin observation

Animals were euthanized by intraperitoneal injection of an excess amount of pentobarbital sodium (100 mg/kg) at the experimental endpoint, and then photographs of local skin were taken.

### 3.7 Experimental endpoint

Animals were weighed at the experimental endpoints and the body weight was recorded to calculate the volume of administration. 6 h after dosing, firstly, whole blood was collected from the orbital venous plexus of mice and subjected to blood routine test using an automated Hematology analyzer (Sysmex XS-800i). The specific test steps were as follows: the blood sample in the test tube was thoroughly mixed before testing; the sample was placed under the sampling needle, then injected by pressing the start button, and removed after indicator light of the instrument was off; the instrument started to automatically test the sample and output the results.

Then the animals were euthanized by intraperitoneal injection of pentobarbital sodium. The material and order of dissection were as follows: Spleen was collected and weighed and the weight was recorded. Bilateral inguinal lymph nodes were quick frozen and stored in a refrigerator at -80°C. The lesioned skin tissue collected was first subject to skin image collection, and then soaked in 10% formalin fixative solution for fixation at a 1:10 ratio of tissue to formalin, and subjected to histopathological detection after fixation for 48 h. The details were as shown in Table 13 below.

**Table 13. Experimental endpoint**

| Name | Content | Storage condition |
|---|---|---|
| Peripheral whole blood (100 µL) | Collected 6 h after the last dosing | Room temperature |
| Plasma | 50 µL × 2 | -80°C |
| | 50 µL + protection solution | |
| Spleen | Weighed | NA |
| Lymph nodes | Bilateral axillary and inguinal lymph nodes | -80°C |
| Lesioned skin | 1/2 of the lesioned skin was fixed | Neutral buffered formalin |
| | 1/4 of the lesioned skin was frozen on dry ice for storage | -80°C |
| | Protection solution was added to 1/4 of the lesioned skin | |

### 3.8 Histopathological detection

The skin tissue of the lesioned skin was subjected to dehydration, embedding and sectioning according to the pathology SOP, and the tissue was stained by HE and Masson staining. Histopathological analysis was performed according to the following methods:

### 3.8.1 Dermal thickness

Masson-stained sections were panoramically scanned by using Nanozomer S210 and the scanned images were subjected to quantitative analysis using Visiopharm VIS6.0 software. The degree of fibrosis was currently represented by the dermal thickness after Masson staining.

### 3.8.2 Capillary density score within the dermis layer

The fields of observation was selected in the dermis layer of the lesioned area (the number of fields selected was determined according to the size of the lesioned area) and scored according to the area occupied by capillaries in the area:
0: No capillary formed
1: The proportion of capillaries formed was less than 25%;
2: The proportion of capillaries formed was 25-50%;
3: The proportion of capillaries formed was 50-75%;
4: The proportion of capillaries formed was greater than 75%.

### 3.8.3 Inflammatory cell infiltration score of dermis layer

The fields of observation was selected in the dermis layer of the lesioned area (the number of fields selected was determined according to the size of the lesioned area) and scored according to the degree of inflammatory cell infiltration:
0: No inflammatory cell infiltration:
1: A small amount of focal inflammatory cell infiltration;
2: Scattered diffuse inflammatory cell infiltration:
3: A large amount of diffuse inflammatory cell infiltration;
4: A large amount of agglomerated inflammatory cell infiltration.

### 3.9 Data Analysis

The mean ± SEM was calculated using Graphpad prism 6.0 software. The significance test of difference was performed using the t-test, one-way ANOVA or two-way ANOVA, and difference between two groups with p < 0.05 was considered as significant.

### 4 Experimental results

### 4.1 Body weight changes

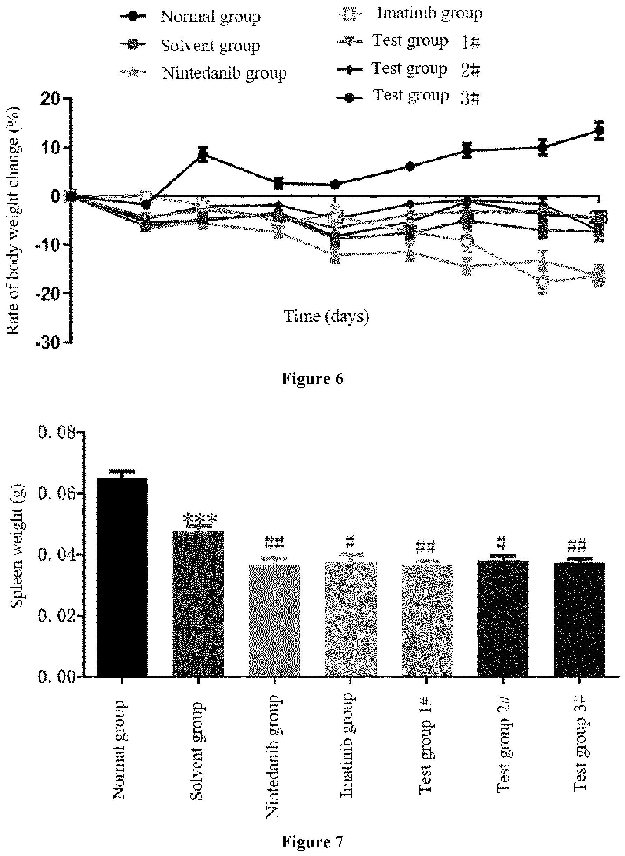

During the experiment, the animal body weight in the normal group showed an increasing trend, and the body weight of Test group 1#, Test group 2# and Test group 3# remained stable and varied within the normal range. Only the body weight of Nintedanib group and Imatinib group showed a slowly decreasing trend after dosing and consistently had significant difference compared with the solvent group over the period from Day 25 to Day 28 (see Figure 5 and Figure 6 below). One animal in Imatinib group died on Day 28, and its body weight on the day of death decreased by 23.7% compared with the body weight before model establishment.

### 4.2 Spleen weight and spleen/body weight ratio

The spleen was collected at the experimental endpoint and weighed to calculate the spleen/body weight ratio (spleen/body weight × 100%), and the results were as shown in Figure 7. The spleen weight of the solvent group was significantly reduced compared with the normal group, and the spleen weight of the Nintedanib group, Imatinib group, Test group 1#, Test group 2# and Test group 3# was also significantly reduced compared with the solvent group.

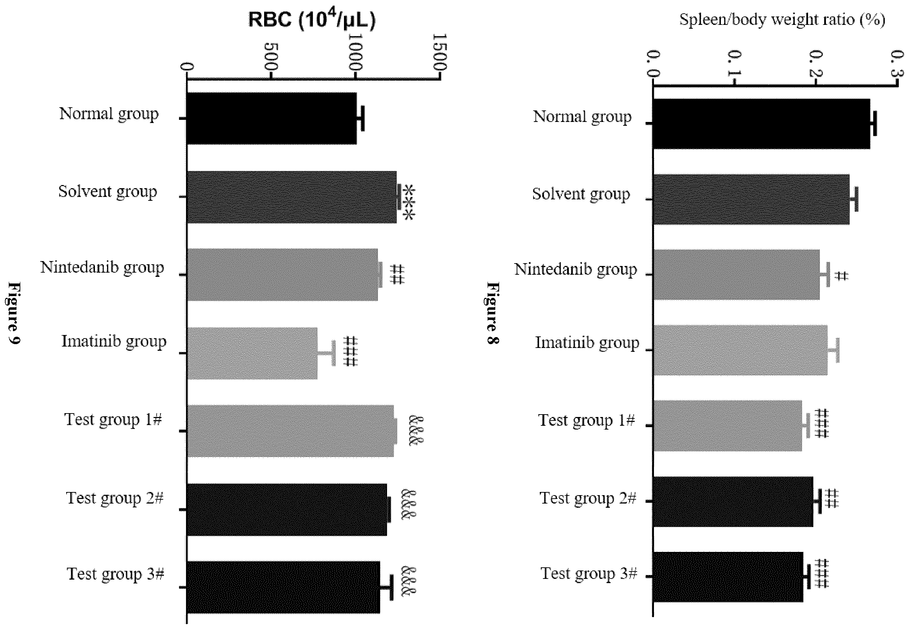

In terms of the spleen/body weight ratio, there was no obvious difference between the solvent group and the normal group, indicating that the model establishment could not change the spleen/body weight ratio of mice. Only the Nintedanib group, Test group 1#, Test group 2# and Test group 3# had significant differences compared with the solvent group (Table 14, Figure 8).

**Table 14. Spleen weight and spleen/body weight ratio**

| Group | Spleen (g) | Spleen/body weight ratio (%) |
|---|---|---|
| Normal group | 0.07±0.00 | 0.27±0.01 |
| Solvent group | 0.05±0.00*** | 0.24±0.01 |
| Nintedanib group | 0.04±0.00^{##} | 0.20±0.01^{#} |
| Imatinib group | 0.04±0.00^{#} | 0.22±0.01 |
| Test group 1# | 0.04±0.00^{##} | 0.18±0.01^{###} |
| Test group 2# | 0.04±0.00^{#} | 0.20±0.01^{##} |
| Test group 3# | 0.04±0.00^{##} | 0.18±0.01^{###} |

| | | |
|---|---|---|
| ***p < 0.001 vs. normal group, ^{#}p < 0.05, ^{##}p < 0.01 vs. solvent group. | | |

### 4.3 Blood routine test

Peripheral blood was collected at the experimental endpoint for blood routine test. The results showed that Imatinib significantly reduced the content of red blood cells and hemoglobin in peripheral blood, which was also consistent with the symptoms of anemia in this group of animals at the experimental endpoint. In addition, Imatinib significantly reduced the content of white blood cells (including lymphocytes and neutrophils) in peripheral blood, and had a trend of reducing monocytes, but there was no significant difference.

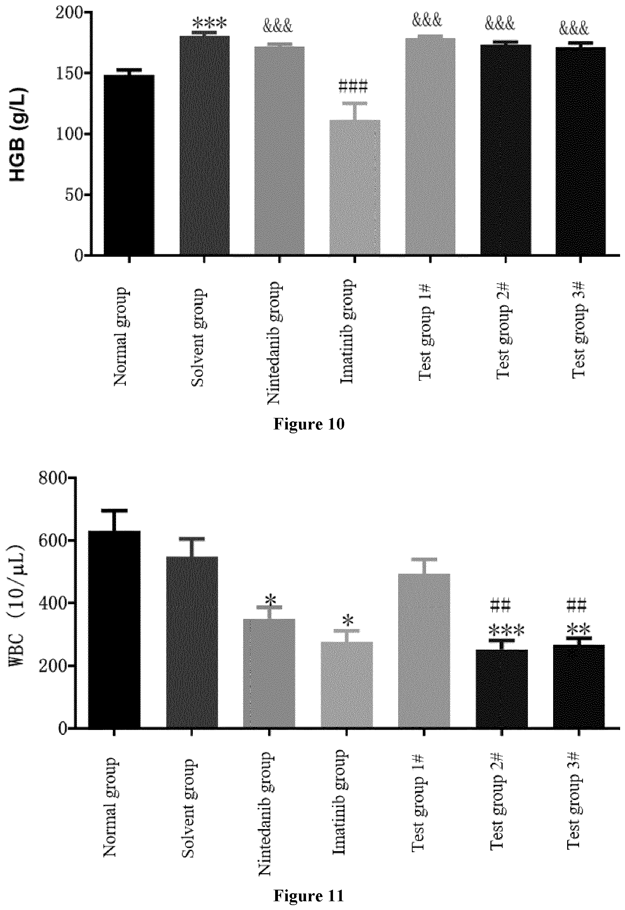

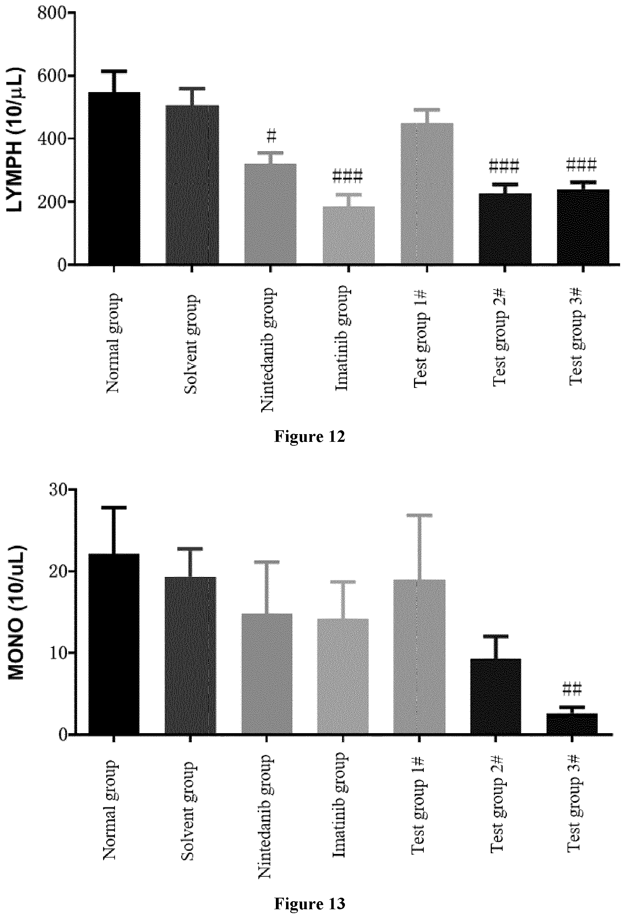

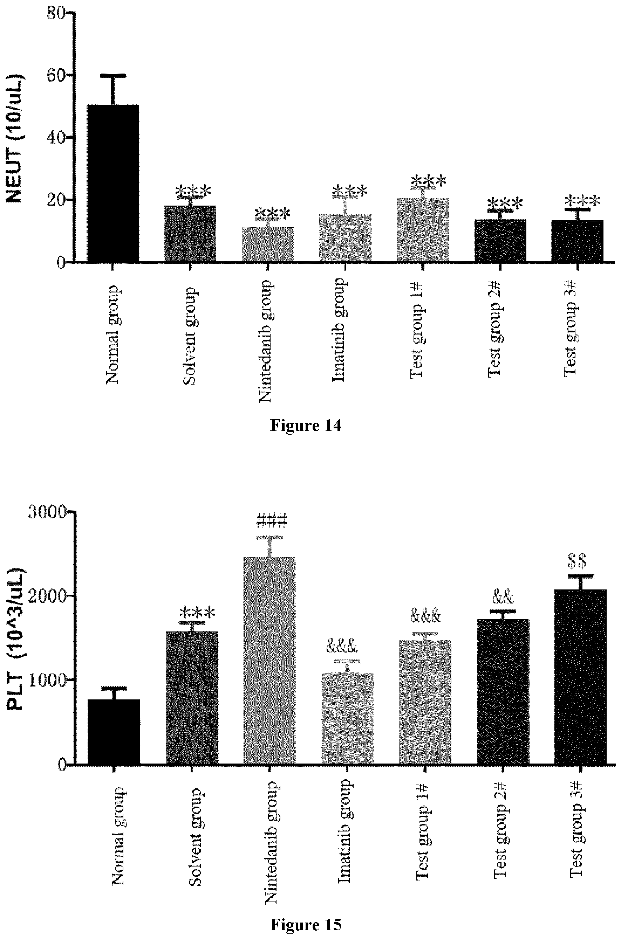

Test group 3# had significant differences in reducing lymphocytes, neutrophils and monocytes compared with the solvent group, indicating that the treatment at the dose of Test group 3# (100 mg/kg) had the effect of reducing inflammation, which was consistent with the pathological results (Figure 9 to Figure 15).

### 4.4 Skin pathological staining results

Systemic sclerosis (SSc) is an autoimmune disease that is characterized by inflammation, vascular lesions, and fibrosis of the skin and organs.

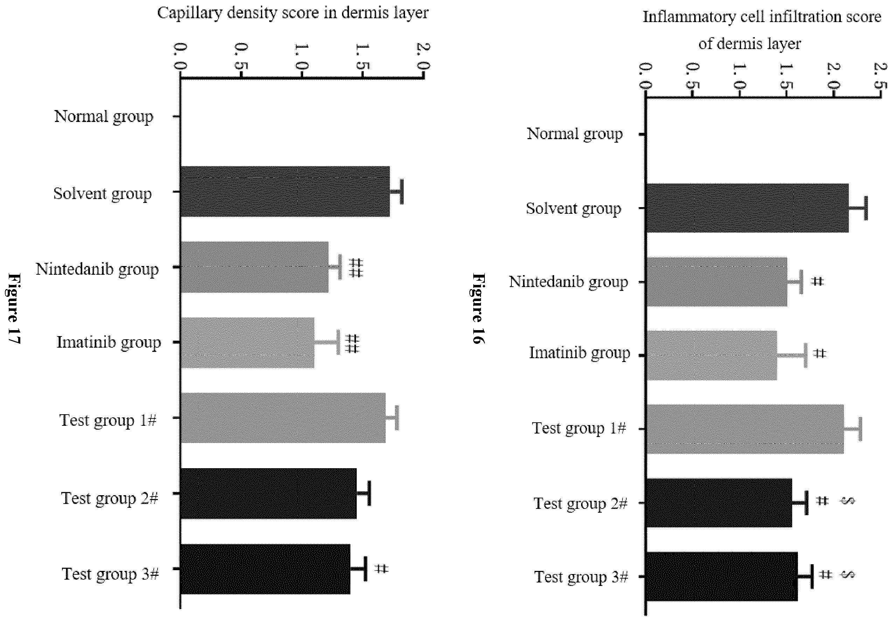

In this example, bleomycin (BLM) was used to establish a mouse fibrosis model. The pathological HE staining results showed significant inflammatory cell infiltration and subdermal capillary hyperplasia compared with the solvent group and the normal group. The Masson staining results showed that the animals in the solvent group had a stronger degree of skin fibrosis, and their dermal thickness also increased significantly. Treatment of Nintedanib and Imatinib could visibly reduce inflammatory cell infiltration and subdermal capillary hyperplasia of the lesioned skin, and Test group 2# and Test group 3# could significantly reduce inflammatory cell infiltration of the lesioned skin and subdermal capillary hyperplasia. According to the statistical results of dermal thickness, the effect for three different doses of the substance to be tested (Nepicastat) to reduce dermal thickness was dose-dependent, and the higher the dose, the more obvious the result, but there was no significant difference compared with the model group. Test group 3# had a better effect on ameliorating skin fibrosis compared with Test group 1# and Test group 2# (Figure 16 to Figure 18).

It could be seen from the above experimental results that Test group 2# and Test group 3# could reduce the number of increased inflammatory cells in peripheral blood after bleomycin stimulation, reduce the inflammatory cell infiltration and capillary hyperplasia in the dermis layer of the lesioned skin. Especially, the dose of 100 mg/kg corresponding to Test group 3# had the best effect, and 100 mg/kg could also reduce the degree of fibrosis of the lesioned skin.

### Example 6: Effect of DBH inhibitors on bovine IRBP R16-induced experimental autoimmune uveoretinitis (EAU) in Lewis rats

### 1. Materials and instruments

**Table 15. Reagents**

| Reagent | Supplier | Batch No. |
|---|---|---|
| PEG400 | Sinopharm Chemical Reagent Co., Ltd. | 20190806 |
| Normal saline | Jiangxi Kelun Pharmaceutical Co, Ltd. | C19070906 |
| 30% Solutol HS15 | Sigma | BCCB9630 |
| Dexamethasone | Shanghai Sine Pharmaceutical Laboratories Co., Ltd. | 015191109 |
| 0.5% Sodium carboxymethylcellulose | Sinopharm Chemical Reagent | 30036365 (Cat No.) |
| IRBP Peptide R16 | GL Biochem Co., Ltd | P191203-TL051092 |
| Complete Freund's adjuvant | Sigma-Aldrich | SLCD6299 |
| *Mycobacterium tuberculosis* H37Ra | Difco Detroit, MI, USA | 231141 (Cat No.) |
| Zoletil 50 | Virbac S.A. | BN 6ALU (Cat No.) |
| Davidson's Fixative | PHYGENE | PH0975 |

**Table 16. Instruments**

| Instrument | Manufacturer | Type |
|---|---|---|
| Tissue processor | Fisher/Thermo | A78400006 |
| Tissue embedding instrument | Fisher/Thermo | B64100010 |

### 2. Preparation and storage of drug solution

Substance to be tested - Nepicastat solution: taking the preparation of 10 mg/mL Nepicastat solution as an example: 40 mg of Nepicastat was weighed and put into a brown sample vial, and 0.8 mL of PEG400 was added. The vial was vortexed on a vortex, ultrasonicated for 15 minutes, and heated in a water bath at 40°C for 15 minutes to form a suspension. Then 0.4 mL of 30% Solutol HS15 was added and the suspension was vortexed. Then 2.8 mL of normal saline was added and the suspension was thoroughly vortexed to form a solution. The solution was freshly prepared every day.

Reference substance - Dexamethasone solution: Dexamethasone was suspended at a concentration of 0.04 mg/mL in a solution of sodium carboxymethylcellulose at a mass concentration of 0.5%. The solution was freshly prepared every day.

IRBP R16 solution: Bovine IRBP R16 polypeptide was dissolved in normal saline to a final concentration of 300 µg/mL.

Complete Freund's adjuvant (CFA): 15 mg of *Mycobacterium tuberculosis* H37Ra was mixed with 10 mL of CFA to a final concentration of 2.5 mg/mL (CFA itself contained 1.5 mg/mL of H37Ra, and 15 mg of H37Ra was added to 10 mL of CFA, resulting in a final concentration of 2.5 mg/mL).

Preparation of emulsion: the emulsion was prepared by a process of manual mixing. First, two 10 mL syringes were used, one of which was used to aspirate 4 mL of 300 µg/mL IRBP R16 solution and connected to a three-way plug valve, while ensuring all air bubbles to be removed. Then a syringe containing 4 mL of CFA was connected and mixing was quickly started. Mixing was done manually by pushing the plunger back and forth for 5 minutes. Finally, the other10 mL syringe was used to aspirate 8 mL of emulsion, then a large caliber needle (e.g., 18 g) was attached, which was inserted into the end of a 1 mL syringe to aliquot the emulsion into ten 1 mL syringes, while the plunger could be pushed back and forth when the needle is attached to the syringe, while ensuring no air bubble was present. The emulsion was used within 3 hours after preparation.

### 3. Experimental methods

The test rats were 6 to 8 week old female Lewis rats purchased from Beijing Charles River Laboratory Animal Technology Co., Ltd., weighing about 180 to 220 g, and were specific pathogen free (SPF) at the beginning of the experiment. The mice were housed in a room at room temperature (20 to 26°C, relative humidity of 40%-70%) (2 to 4 mice per cage) with a 12/12-hour light-dark cycle. All experimental protocols were approved by the IACUC (Institutional Animal Care and Use Committee) of PharmaLegacy. The test rats were adapted to the housing conditions in the laboratories of PharmaLegacy for 7 days before the experiment.

### (1) Immunization of rats with IRBP R16 emulsion

On Day -1 (i.e., the day before immunization, hereinafter), 60 rats were randomly divided into 6 groups (n=10) based on body weight (see Table 18). On Day 0, rats in Groups 2 to 6 were anesthetized with isoflurane, followed by subcutaneous injection of a total of 200 mL of emulsion resulting from IRBP R16 and CFA emulsified at a 1:1 v/v ratio, on both thighs (50 µL at each site) and tail head (100 µL), respectively.

Wherein, Day 0 referred to the day of immunization. The body weight of each rat was monitored twice a week after immunization.

### (2) Clinical evaluation of EAU in rats

From Day 0 after immunization, the eyes of rats were checked daily with a flashlight by gently opening the upper and lower eyelids of the test rats, and the incidence of disease was recorded. Clinical symptoms were scored in a blind manner from the onset of disease to the end of the study according to the criteria listed in Table 17.

**Table 17. Clinical score of EAU in rats**

| Score | Criteria |
|---|---|
| 0 | No disease; translucent eyes with light reflection (red reflex) |
| 0.5 (trace amount) | Vasodilation in iris |
| 1 | Vascular filling in iris; abnormal pupillary constriction |
| 2 | Blurred anterior chamber; weakened red reflex |
| 3 | Moderately opaque anterior chamber but pupils still visible; dark red reflex |
| 4 | Anterior chamber opacification, blurred pupils; absence of red reflex; exophthalmos |

### (3) Dosing regimen

Dosing started from Day 0 accurately according to the animal body weight for a total of 16 days. The dosing regimen for test rats was shown in Table 18.

**Table 18. Dosing regimen**

| Group | Drug | Number of rats | Route of administrat ion | Concentration of stock solution (mg/mL) | Dose | | Frequency of administrat ion |
|---|---|---|---|---|---|---|---|
| | | | | | mL/kg | mg/kg | |
| 1 | Blank control^{a} | 10 | PO | N/A | 10 | N/A | QD × 16 days |
| 2 | Model control^{a} | 10 | PO | N/A | 10 | N/A | QD × 16 days |
| 3 | Dexamethas one^{b} | 10 | PO | 0.04 | 10 | 0.4 | QD × 16 days |
| 4 | Nepicastat (25 mpk) | 10 | PO | 2.5 | 10 | 25 | QD × 16 days |
| 5 | Nepicastat (50 mpk) | 10 | PO | 5 | 10 | 50 | QD × 16 days |
| 6 | Nepicastat (100 mpk) | 10 | PO | 10 | 10 | 100 | QD × 16 days |

| | | | | | | | |
|---|---|---|---|---|---|---|---|
| a: 20% PEG400 + 10% (30% Solutol HS15) + 70% normal saline b: 0.5% sodium carboxymethylcellulose. | | | | | | | |

### (4) Statistical analysis of experimental results

The results were expressed as "mean ± standard error". Graphpad Prism or SPSS was used for statistical analysis, and p<0.05 was considered statistically significant.

### 4. Experimental results

### (1) Rat body weight

The body weight changes of the test rats were specifically as shown in Table 19 below, represented as mean and standard error, respectively.

**Table 19. Rat body weight (g)**

| Mean | Day -1 | Day 2 | Day 6 | Day 9 | Day 13 | Day 16 |
|---|---|---|---|---|---|---|
| Blank control | 195.89 | 198.20 | 209.00 | 215.42 | 216.80 | 221.80 |
| Model control | 186.99 | 192.94 | 200.05 | 204.24 | 209.85 | 210.04 |
| Dexamethasone | 188.46 | 174.65 | 166.65 | 164.05 | 162.10 | 161.29 |
| Nepicastat (25 mpk) | 188.31 | 193.95 | 204.67 | 207.81 | 214.28 | 219.00 |
| Nepicastat (50 mpk) | 189.34 | 194.04 | 202.28 | 206.07 | 216.20 | 218.42 |
| Nepicastat (100 mpk) | 189.92 | 194.02 | 205.97 | 214.36 | 223.98 | 227.40 |

| Standard error | Day -1 | Day 2 | Day 6 | Day 9 | Day 13 | Day 16 |
|---|---|---|---|---|---|---|
| Blank control | 2.77 | 2.32 | 1.97 | 2.84 | 2.62 | 3.28 |
| Model control | 2.49 | 1.87 | 2.47 | 2.33 | 2.98 | 2.84 |
| Dexamethasone | 2.41 | 1.66 | 2.19 | 2.29 | 3.13 | 3.27 |
| Nepicastat (25 mpk) | 2.55 | 2.13 | 2.94 | 2.31 | 2.50 | 3.23 |
| Nepicastat (50 mpk) | 2.61 | 2.21 | 2.14 | 3.36 | 3.39 | 2.81 |
| Nepicastat (100 mpk) | 2.72 | 3.82 | 3.38 | 3.85 | 3.28 | 3.86 |

### (2) Weight change rate

**Table 20. Weight change rate (%)**

| Mean | Day -1 | Day 2 | Day 6 | Day 9 | Day 13 | Day 16 |
|---|---|---|---|---|---|---|
| Blank control | 0.00% | 3.47% | 7.84% | 9.71% | 11.59% | 13.89% |
| Model control | 0.00% | 3.25% | 7.06% | 9.26% | 12.23% | 12.36% |
| Dexamethasone | 0.00% | -7.26% | -11.53% | -12.92% | -13.99% | -14.44% |
| Nepicastat (25 mpk) | 0.00% | 3.25% | 7.06% | 9.26% | 12.23% | 12.36% |
| Nepicastat (50 mpk) | 0.00% | 2.53% | 6.90% | 8.83% | 14.24% | 15.41% |
| Nepicastat (100 mpk) | 0.00% | 2.11% | 8.45% | 12.84% | 17.96% | 19.77% |

| Standard error | Day -1 | Day 2 | Day 6 | Day 9 | Day 13 | Day 16 |
|---|---|---|---|---|---|---|
| Blank control | 0.00% | 0.61% | 0.88% | 0.78% | 0.76% | 0.81% |
| Model control | 0.00% | 0.72% | 1.24% | 0.60% | 0.75% | 0.95% |
| Dexamethasone | 0.00% | 0.92% | 0.91% | 0.91% | 1.28% | 1.18% |
| Nepicastat (25 mpk) | 0.00% | 0.72% | 0.84% | 0.87% | 1.20% | 1.06% |
| Nepicastat (50 mpk) | 0.00% | 0.61% | 0.81% | 0.87% | 1.48% | 1.05% |
| Nepicastat (100 mpk) | 0.00% | 0.97% | 0.88% | 0.84% | 0.89% | 1.46% |

### (3) Clinical score

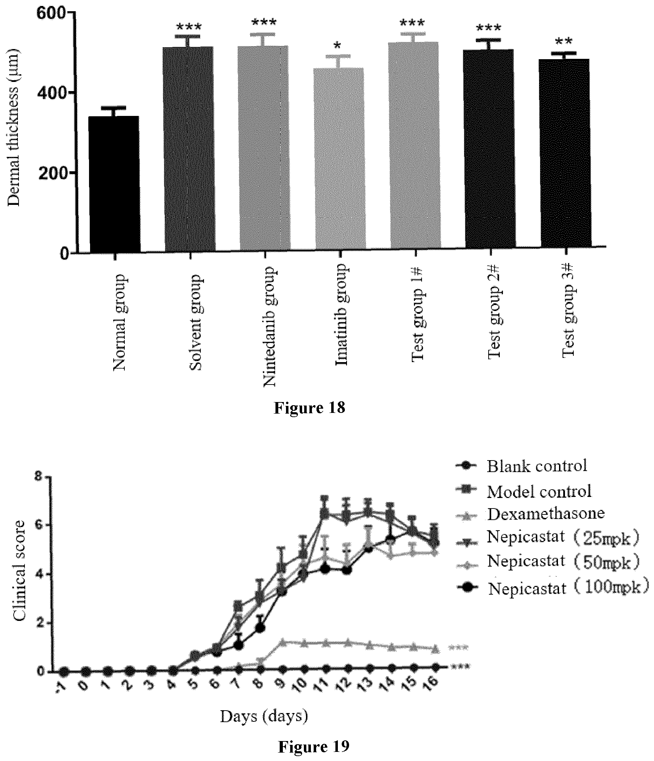

See Figure 19 and Table 21.

**Table 21. Area under the curve (AUC) of clinical score**

| | Blank control | Model control | Dexamethasone | Nepicastat (25 mpk) | Nepicastat (50 mpk) | Nepicastat (100 mpk) |
|---|---|---|---|---|---|---|
| AUC | 0 | 49.60 | 7.68 | 45.30 | 39.65 | 37.65 |
| Standard error | 0 | 3.80 | 0.85 | 4.27 | 4.30 | 5.14 |
| Inhibition rate^{a} | N/A | N/A | 84.53% | 8.67% | 20.06% | 24.09% |

| | | | | | | |
|---|---|---|---|---|---|---|
| a: Inhibition rate = [AUC (model control) - AUC (test group)]/AUC (model control), the test group included the Dexamethasone group and the Nepicastat groups. | | | | | | |

### (4) Incidence rate

**Table 22. Incidence rate (%)**

| Day | Blank control | Model control | Dexamethasone | Nepicastat (25 mpk) | Nepicastat (50 mpk) | Nepicastat (100 mpk) |
|---|---|---|---|---|---|---|
| Day -1 | 0 | 0 | 0 | 0 | 0 | 0 |
| Day 0 | 0 | 0 | 0 | 0 | 0 | 0 |
| Day 1 | 0 | 0 | 0 | 0 | 0 | 0 |
| Day 2 | 0 | 0 | 0 | 0 | 0 | 0 |
| Day 3 | 0 | 0 | 0 | 0 | 0 | 0 |
| Day 4 | 0 | 0 | 0 | 0 | 0 | 0 |
| Day 5 | 0 | 80 | 0 | 70 | 70 | 80 |
| Day 6 | 0 | 100 | 0 | 100 | 100 | 80 |
| Day 7 | 0 | 100 | 10 | 70 | 100 | 50 |
| Day 8 | 0 | 100 | 20 | 100 | 100 | 100 |
| Day 9 | 0 | 100 | 100 | 100 | 100 | 100 |
| Day 10 | 0 | 100 | 100 | 100 | 100 | 100 |
| Day 11 | 0 | 100 | 90 | 100 | 100 | 100 |
| Day 12 | 0 | 100 | 90 | 100 | 100 | 100 |
| Day 13 | 0 | 100 | 90 | 100 | 100 | 100 |
| Day 14 | 0 | 100 | 90 | 100 | 100 | 100 |
| Day 15 | 0 | 100 | 90 | 100 | 100 | 100 |
| Day 16 | 0 | 100 | 90 | 100 | 100 | 100 |

The increase in the clinical score of EAU could prove the successful establishment of a rat uveitis model. Treatment with Nepicastat could moderately ameliorate the symptoms of uveitis and reduced the EAU clinical score and clinical score AUC, but this effect was not statistically significant.

The inhibition rates Nepicastat on clinical score AUC at doses of 25, 50 and 100 mg/kg were 8.67%, 20.06% and 24.09%, respectively. As a positive control, treatment with Dexamethasone significantly reduced the clinical score and clinical score AUC of uveitis, and the inhibition rate of clinical score AUC was 84.53%.

### Example 7: Effects of DBH inhibitors on NMO-IgG-induced neuromyelitis optica (NMO) in mice

### 1. Materials and instruments

**Table 23. Reagents**

| Reagent | Supplier | Batch No. |
|---|---|---|
| PEG400 | Sinopharm Chemical Reagent Co., Ltd. | 20190806 |
| Normal saline | Jiangxi Kelun Pharmaceutical Co, Ltd. | C19070906 |
| 30% Solutol HS15 | Sigma | BCCB9630 |
| Dexamethasone | Shanghai Sine Pharmaceutical Laboratories Co., Ltd. | 015191109 |
| 5% Chloral hydrate | LEAGENE | 0415A19 |

**Table 24. Instruments**

| Instrument | Manufacturer | Type |
|---|---|---|
| RWD brain stereotaxic apparatus | RWD Life Science Co., Ltd. | 68025 |
| KD Scientific syringe pump | Kd Scientific | legato130 |
| Hamilton trace syringe | Hamilton | Gastight#1702 |
| Disposable surgical suture needles with thread | Ningbo Medical Needle Co., Ltd. | Cutting needle 3/0 single needle |

### 2. Preparation and storage of drug solution

Substance to be tested - Nepicastat solution: taking the preparation of 5mg/mL Nepicastat solution as an example: 50mg of Nepicastat was weighed and put into a brown sample vial, and 1.98 mL of PEG400 was added. The vial was vortexed on a vortex, ultrasonicated for 15 minutes, and heated in a water bath at 40°C for 15 minutes to form a suspension. Then 0.99 mL of 30% Solutol HS15 was added and 2.97 mL of suspension was vortexed. Then 6.93 mL of normal saline was added and the suspension was thoroughly vortexed to form a solution. The solution was freshly prepared every day.

Reference substance - Dexamethasone solution: Dexamethasone was dispersed in normal saline at a concentration of 0.1 mg/mL. The solution was freshly prepared every day.

Reference substance - Tanshinone IIa solution: Tanshinone IIa was dissolved in PBS containing 2% DMSO, at a concentration of 0.0736 mg/mL.

### 3. Experimental methods

The test mice were female C57 mice purchased from Beijing Charles River Laboratory Animal Technology Co., Ltd., weighing about 20 to 22 g, and were specific pathogen free at the beginning of the experiment.

### (1) Steps of model establishment

1) C57 female mice (6 to 8 weeks old) were anesthetized with intraperitoneal injection of 5% chloral hydrate (70 µL/10 g).
2) Fur of the top of head was removed to expose the scalp. The mouse head was fixed on the brain stereotaxic apparatus, keeping the top of the skull horizontal. The scalp was disinfected with iodophor.
3) The scalp was cut lengthwise for about 0.5 cm to search for the anterior and posterior fontanelle areas. The stereotaxic caliper was rotated to move the needle tip of trace syringe to the anterior and posterior fontanelle areas, and the mouse skull was kept horizontal by observing the distance from Bregma and Lambda to the needle tip. After horizontal adjustment, the needle tip was moved to Bregma and then moved 2 mm to the right to reach the position.
4) The position on the surface of the mouse skull was marked and a hole was drilled at this point with a skull drill. During drilling, blood vessels should be avoided and drilling should be continued carefully to avoid damaging the duramater and brain tissue.
5) The trace syringe was vertically introduced into the mouse brain tissue using the stereotaxic apparatus with a vertical needle depth of 3 mm. The syringe was kept in place and maintained for 5 min.
6) The syringe pump was turned on, the Infuse Only mode was selected, and the solution (2 µL NMO-IgG + 3 µL human complement + 5 µL PBS/drug, mixed and pipetted well in advance, and placed on ice) was injected at a speed of 1 µL/min.
7) After the injection was completed, the syringe was maintained for 10 min. The syringe was slowly pulled upward by rotating the caliper, and maintained for 5 min after moving 1 mm upward, until the syringe was completely pulled out. The skin was sutured and disinfected again with iodophor.

### (2) Dosing regimen

Dosing started 3 days before model establishment and lasted until 7 days after model establishment, for 10 consecutive days. The specific dosing regimen was as shown in Table 25 below.

**Table 25. Dosing regimen**

| Gro up | Drug | Number of rats | Route of administ ration | Concentration of stock solution (mg/mL) | Dose | | Frequency of administra tion |
|---|---|---|---|---|---|---|---|
| | | | | | mL/kg | mg/kg | |
| 1 | Solvent | 15 | PO | N/A | 10 | N/A | QD × 10 days |
| 2 | Tanshinone II | 15 | IP | 0.0736 | 10 | 736 µg/mL | QD × 10 days |
| 3 | Dexamethas one | 15 | IP | 0.1 | 10 | 1 mg/kg | QD × 10 days |
| 4 | Nepicastat (50 mpk) | 15 | PO | 5 | 10 | 50 | QD × 10 days |
| 5 | Nepicastat (100 mpk) | 15 | PO | 10 | 10 | 100 | QD × 10 days |

### (3) Sample collection

On Day 8 after model establishment (i.e., the day after the last dose), the experimental mice were euthanized and brain tissue was collected for frozen sections. Then, the immune response area of astrocyte markers in each group of mice was determined and evaluated (see the reference Ye Gong et al., Journal of Neuroinflammation, 17(1). doi:10.1186/s12974-020-01874-6 for experimental methods).

### 4. Experimental results

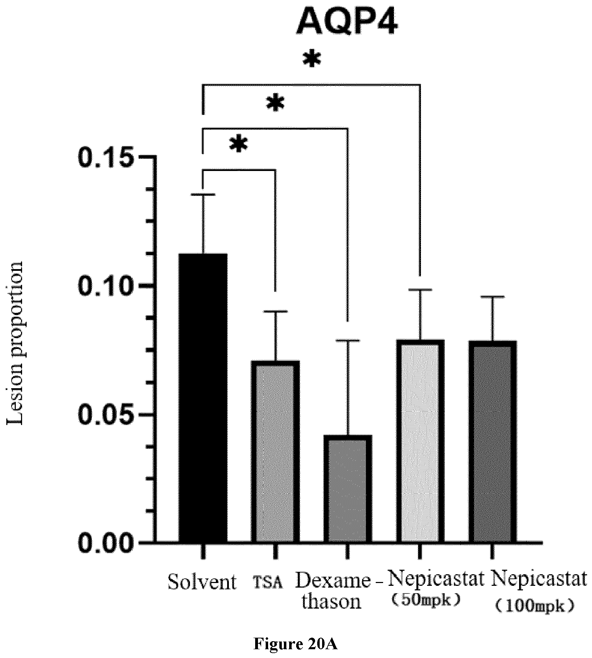

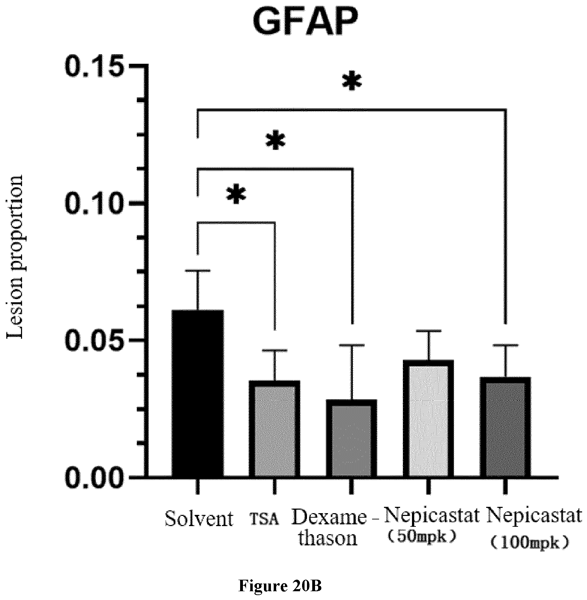

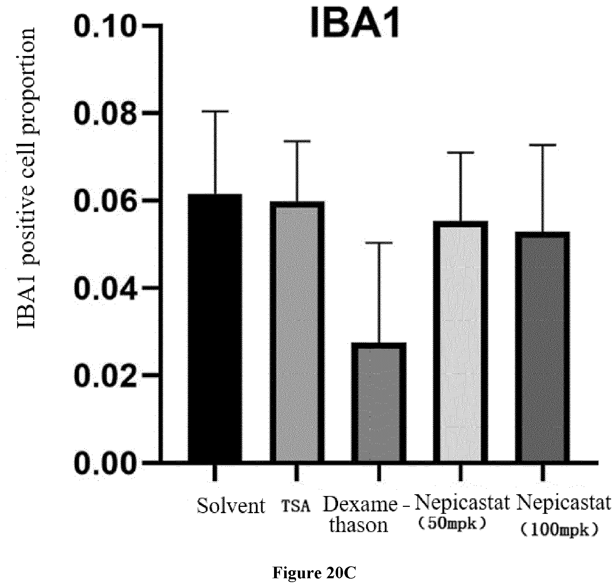

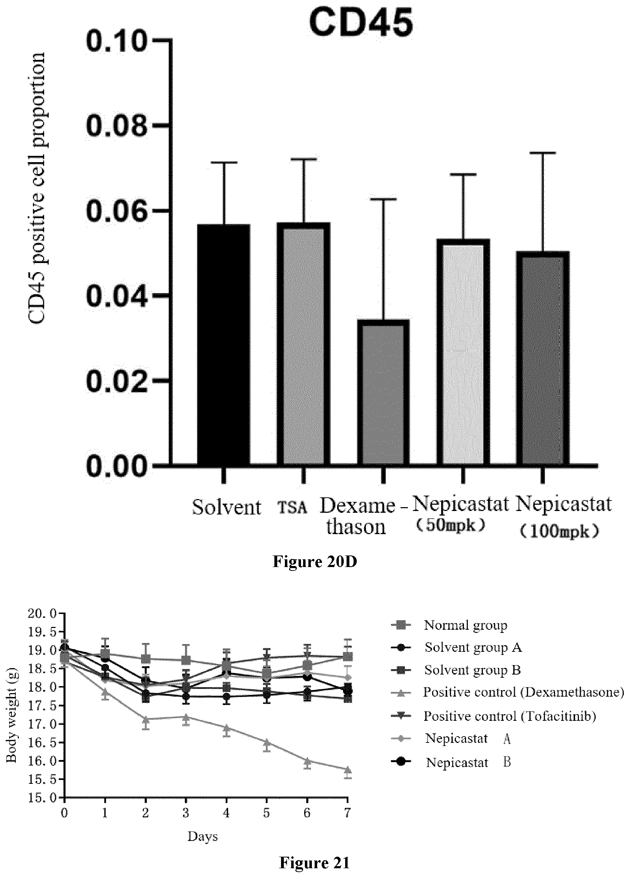

Figures 20A to 20D showed that in terms of the lesion ratio of AQP4 and GFAP, the Nepicastat groups could significantly reduce the lesion ratio compared with the control group, and the effect was less than that of the positive drug group. However, there was no significant effect on the immune parameters CD45 and IBA1. Therefore, Nepicastat had a certain inhibitory effect on NMO-IgG-induced neuromyelitis optica in mice.

### Example 8: Therapeutic effect of DBH inhibitors on Imiquimod (IMQ)-induced psoriasis

### 1. Materials and instruments

**Table 26. Reagents**

| Reagent | Supplier | Catalog No. |
|---|---|---|
| 5% Imiquimod cream | Aldara, 3M Pharmaceuticals | / |
| Dexamethasone cream | Sanjun Pharmaceuticals Co., Ltd. | H44024170 |
| Tofacitinib citrate | Dalian Meilunbio Co., Ltd. | MB3358 |
| PEG400 | Sigma | 202398 |
| Solutol HS15 | Sigma | 42966 |
| EtOH | Shanghai Aladdin Biochemical Technology Co., Ltd. | A500737-0500 |
| Cremophor EL | Sigma | C5135 |
| Poloxamer 188 | Sigma | 9003-11-6 |

**Table 27. Instruments**

| Instrument | Type |
|---|---|
| Electronic balance | MFC: 9092250 |
| Analytical balance | SECURA225D-1CN+YDP20-0CEV1 |
| Centrifuge | Eppendorf_5424 |

**Table 28. Experimental animals**

| | |
|---|---|
| Animal species and strain: | Balb/c mice |
| Breeder/supplier: | Beijing Charles River Laboratory Animal Technology Co., Ltd. |
| Sex, age: | Female, 7-8 weeks old |
| Experimental institution: | WuXi AppTec Pharmaceutical Technology Co., Ltd. (Nantong) |
| Adaptation period: | 7 days |
| Room: | SPF grade |
| Room temperature: | 20-26°C |
| Relative humidity: | 40-70% |
| Light-dark cycle: | 12/12-hour light/dark cycle |
| Housing density: | 5 animals/cage |
| Food and water: | Free access to food and water |

All experiments performed in this protocol were approved by the Institutional Animal Care and Use Committee (IACUC) of WuXi AppTec.

### 2. Preparation and storage of drug solution

Substance to be tested - Nepicastat solution for oral administration: 9.9 mL of Nepicastat solution with a concentration of 5 mg/mL for oral administration was prepared: 50 mg of Nepicastat was weighed and 1.98 mL of PEG400 was added. The mixture was ultrasonicated for 15 minutes, heated in a water bath at 40°C for 15 minutes, and vortexed to form a suspension. 0.99 mL of 30% Solutol HS15 was added to 1.98 mL of Nepicastat suspension and vortexed to form a suspension. 6.93 mL of normal saline was added to 2.97 mL of Nepicastat suspension and vortexed to form a solution.

Substance to be tested - Nepicastat solution for topical administration: 1 mL of Nepicastat (50 mg/mL) for external use was prepared: 50.13 mg of Nepicastat was weighed and 100 µL of PG was added. The mixture was stirred at 45°C for 10 minutes to obtain a homogeneous opaque suspension. Then 200 µL of ethanol was added and the mixture was stirred at 45°C for 5 minutes to obtain a homogeneous opaque suspension. Then 200 µL of Cremophor EL was added and the mixture was stirred at 45°C for 5 minutes to obtain a homogeneous opaque suspension. Then 500 µL of 5% Poloxamer 188 was added to the suspension and the mixture was stirred at 45°C for 30 minutes to obtain a clear solution.

Reference substance - Tofacitinib citrate solution: Tofacitinib citrate was dissolved in dimethyl sulfoxide to obtain a solution with a concentration of 5 mg/mL. 50 µL of the above solution was added to IMQ ointment for topical application.

### 3. Experimental methods

### (1) Shaving fur

All mice were shaved with a pet shaver to get an area of 2×3 cm on the back one day before the start of the experiment.

### (2) Grouping

65 animals were randomly divided into groups according to body weight, and the dosing regimen was as follows.

**Table 29. Grouping and dosing regimen**

| Group | Number of mice | Experiment al drug | Topical induction | Treatme nt | Dose (mg/kg) | Dosing regimen | |
|---|---|---|---|---|---|---|---|
| | | | | | | Route | Frequenc y |
| 1 | 5 | Normal group | No | N/A | N/A | N/A | N/A |
| 2 | 10 | Solvent group A | 62.5 mg 5% IMQ QD × 7 days | 10 mL/kg | N/A | Oral administratio n | QD × 7 days |
| 3 | 10 | Solvent group B | 62.5 mg 5% IMQ QD × 7 days | 40 µL/mou se | N/A | Topical administratio n | QD × 7 days |
| 4 | 10 | Positive control (Dexameth asone) | 62.5 mg 5% IMQ QD × 7 days | 70 mg ointme nt/mous e | 2 | Topical administratio n | QD × 7 days |
| 5 | 10 | Positive control (Tofacitini b) | 62.5 mg 5% IMQ QD × 7 days | 5 mg/mL | 12.5 | Topical administratio n | QD × 5 days |
| | | | | 50 µL/mou se | | | BID × 2 days |
| 6 | 10 | Nepicastat A | 62.5 mg 5% IMQ QD × 7 days | 10 mL/kg | 50 | Oral administratio n | QD × 7 days |
| 7 | 10 | Nepicastat B | 62.5 mg 5% IMQ QD × 7 days | 40 µL/mou se | 100 | Topical administratio n | QD × 7 days |

| | | | | | | | |
|---|---|---|---|---|---|---|---|
| Solvent A: 20% PEG400 + 10% (30% Solutol HS15) + 70% normal saline; Solvent B: 10% PG/20% EtOH/20% Cremophor EL/50% (5% Poloxamer 188 aqueous solution). | | | | | | | |

### (3) IMQ and positive control formulations

Imiquimod (IMQ) cream: 62.5 mg of IMQ cream for each mouse in G2 to G7;
Dexamethasone (DEX) ointment: 70 mg of ointment for each mouse in G4;
Tofacitinib: Tofacitinib was dissolved in dimethyl sulfoxide to 5 mg/mL, and 50 µL was added to IMQ cream for daily treatment.

### (4) IMQ sensitization and dosing

Mice in Groups 2 to 7 received daily topical doses of 62.5 mg of IMQ cream on their shaved back from Day 0 to Day 7.

Dosing of the test compounds and reference substance was carried out according to Table 29 for 7 consecutive days.

### (5) Disease assessment

To assess the severity of inflammation of skin on the back, the skin on the back was scored according to Table 30. Erythema, scales and thickening were scored independently on a scale of 0 to 3, 0, none; 1. mild; 2. moderate; 3. obvious/severe.

**Table 30. Clinical scoring parameters**

| Clinical scoring parameters | |
|---|---|
| Erythema | None (0), mild (1), moderate (2), severe (3) |
| Thickness | None (0), mild (1), moderate (2), severe (3) |
| Scales | None (0), mild (1), moderate (2), severe (3) |

### (6) End of experiment and sample collection

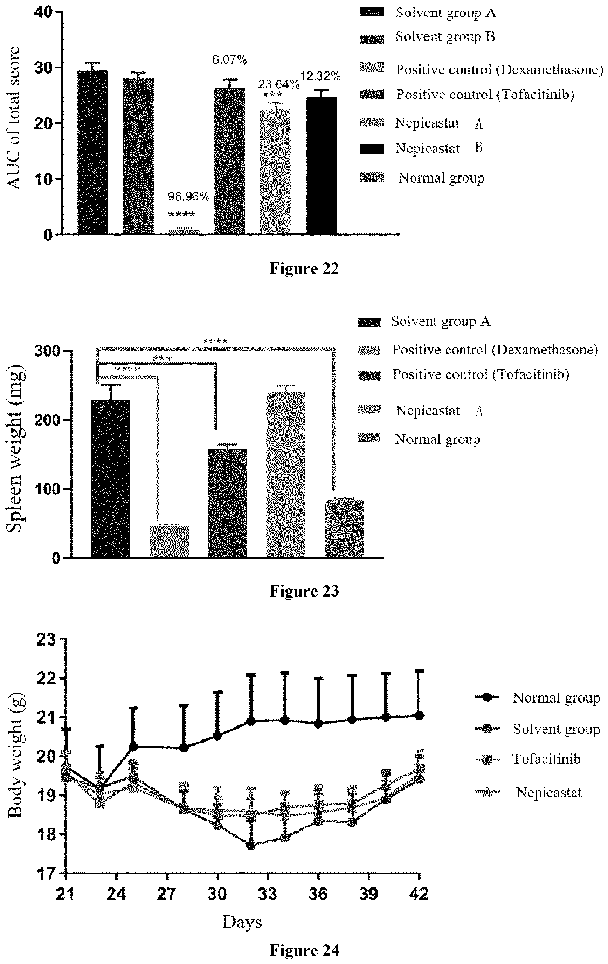

At the end of the experiment, mice were euthanized by CO₂ inhalation:
1) Blood was collected by cardiac puncture. Plasma was processed and divided into 3 aliquots, with 1 aliquot in protection solution and 2 aliquots quick frozen;
2) 5 samples of skin on the back in each group were collected and put into protection solution, 5 samples of skin on the back were placed into PFA, and 5 samples of skin on the back were quick frozen;
3) Lymph nodes were collected and quick frozen;
4) Spleen samples were collected and weighed (Figure 23).

### (7) Data statistics

GraphPad Prism6.0 software was used to perform statistical analysis of data by one-way ANOVA.

### 4. Research results

### (1) Body weight

Body weight was monitored daily throughout the study. Continuous use of IMQ cream could reduce the mean body weight of the solvent group. In addition to this, weight loss was more dramatic in the Dexamethasone treatment group (G4). However, there was no statistically significant difference between the treatment and solvent groups (Figure 21).

### (2) Clinical score

Erythema, scales and thickness of the skin on the back were scored daily. In addition, the sum of the three (erythema + thickness + scales) represented the total score. The Dexamethasone treatment group had a significant inhibitory effect on inflammation, suggesting successful establishment of the IMQ cream-induced psoriasis model.

The Nepicastat oral administration group inhibited erythema and thickness from Day 5 to Day 7. The Nepicastat topical administration group inhibited erythema from Day 4 to Day 7. Tofacitinib showed efficacy after doubling the dose. Nepicastat treatment did not affect the progression and incidence of the disease, but showed efficacy at the later stages of the disease course (Day 5 to Day 7). The area under curve (AUC) was calculated based on the total clinical score curve in each group of animals (Figure 22).

The inhibition rate was calculated according to the following formula: Inhibition rate = [AUC (solvent) - AUC (treatment)] / AUC (solvent) × 100%. Wherein, solvent referred to the solvent group corresponding to the route of administration.

The inhibition rate of Dexamethasone treatment group was 96.96%. The Nepicastat 50 mpk oral treatment group had a significant inhibitory effect on the clinical score of the skin, with an inhibition rate of 23.64%. However, The Nepicastat 100 mpk topical treatment group had no significant efficacy.

**Table 31. Results of erythema score**

| | Day 0 | Day 1 | Day 2 | Day 3 | Day 4 | Day 5 | Day 6 | Day 7 |
|---|---|---|---|---|---|---|---|---|
| Normal group | 0 | 0 | 0 | 0 | 0 | 0 | 0 | 0 |
| Solvent group A | 0 | 0.1 | 1.6 | 2 | 2.2 | 2.4 | 2.5 | 2.5 |
| Solvent group B | 0 | 0 | 1.5 | 2 | 2.2 | 2.2 | 2.2 | 2.3 |
| Positive control (Dexamethasone) | 0 | 0 | 0 | 0.2 | 0.1 | 0.1 | 0 | 0 |
| Positive control (Tofacitinib) | 0 | 0 | 1.4 | 1.9 | 2.2 | 2 | 1.8 | 1.7 |
| Nepicastat A | 0 | 0 | 1.4 | 1.5 | 1.6 | 1.6 | 1.7 | 1.6 |
| Nepicastat B | 0 | 0 | 1.5 | 1.8 | 1.8 | 1.7 | 1.5 | 1.2 |

**Table 32. Results of scales score**

| | Day 0 | Day 1 | Day 2 | Day 3 | Day 4 | Day 5 | Day 6 | Day 7 |
|---|---|---|---|---|---|---|---|---|
| Normal group | 0 | 0 | 0 | 0 | 0 | 0 | 0 | 0 |
| Solvent group A | 0 | 0 | 0 | 0.1 | 0.9 | 2.1 | 2.7 | 2.3 |
| Solvent group B | 0 | 0 | 0 | 0 | 1 | 2.4 | 3 | 2.3 |
| Positive control (Dexamethasone) | 0 | 0 | 0 | 0 | 0.2 | 0.2 | 0.2 | 0.1 |
| Positive control (Tofacitinib) | 0 | 0 | 0 | 0.4 | 0.7 | 1.4 | 1.3 | 1.6 |
| Nepicastat A | 0 | 0 | 0 | 0 | 1.1 | 1.6 | 1.9 | 2.1 |
| Nepicastat B | 0 | 0 | 0 | 0 | 1.2 | 2.5 | 2.4 | 2.1 |

**Table 33. Results of thickness score**

| | Day 0 | Day 1 | Day 2 | Day 3 | Day 4 | Day 5 | Day 6 | Day 7 |
|---|---|---|---|---|---|---|---|---|
| Normal group | 0 | 0 | 0 | 0 | 0 | 0 | 0 | 0 |
| Solvent group A | 0 | 0.9 | 1.1 | 1.6 | 1.8 | 2 | 2 | 2 |
| Solvent group B | 0 | 0.9 | 1.1 | 1.5 | 1.5 | 1.7 | 1.6 | 1.8 |
| Positive control (Dexamethasone) | 0 | 0 | 0 | 0 | 0 | 0 | 0 | 0 |
| Positive control (Tofacitinib) | 0 | 0.8 | 1.4 | 2.2 | 2 | 2.2 | 2 | 1.9 |
| Nepicastat A | 0 | 0.6 | 1.2 | 1.6 | 1.4 | 1.3 | 1.4 | 1.4 |
| Nepicastat B | 0 | 0.9 | 1.3 | 1.5 | 1.5 | 1.3 | 1.3 | 1.4 |

**Table 34. Results of total score of erythema + scales + thickness**

| | Day 0 | Day 1 | Day 2 | Day 3 | Day 4 | Day 5 | Day 6 | Day 7 |
|---|---|---|---|---|---|---|---|---|
| Normal group | 0 | 0 | 0 | 0 | 0 | 0 | 0 | 0 |
| Solvent group A | 0 | 1 | 2.7 | 3.7 | 4.9 | 6.5 | 7.2 | 6.8 |
| Solvent group B | 0 | 0.9 | 2.6 | 3.5 | 4.7 | 6.3 | 6.8 | 6.4 |
| Positive control (Dexamethasone) | 0 | 0 | 0 | 0 | 0.3 | 0.3 | 0.2 | 0.1 |
| Positive control (Tofacitinib) | 0 | 0.8 | 2.8 | 4.5 | 4.9 | 5.6 | 5.1 | 5.2 |
| Nepicastat A | 0 | 0.6 | 2.6 | 3.1 | 4.1 | 4.5 | 5 | 5.1 |
| Nepicastat B | 0 | 0.9 | 2.8 | 3.3 | 4.5 | 5.5 | 5.2 | 4.7 |

5. The objective of this Example was to study the efficacy of the test compounds on an IMQ-induced psoriasis model. The results showed that application of IMQ led to severe skin inflammation. The Nepicastat PO treatment group had a mild therapeutic effect on clinical symptoms compared with the solvent group and the control group.

### Example 9: Therapeutic effect of DBH inhibitors on collagen-induced arthritis (CIA) in mice.

### 1. Experimental materials

Bovine type II collagen, CII, Sichuan University;
Acetic acid, Sigma (St. Louis, MO, USA), Catalog No. A8976;
Complete Freund's adjuvant, Sigma, Catalog No. F5881;
20% PEG400 + 10% (30% Solutol HS15) + 70% normal saline;
Tofacitinib, > 98%, Dalian Meilunbio Co., Ltd. MB3358.

### 2. Test compounds

**Table 35**

| | |
|---|---|
| Compound name | Nepicastat |
| Appearance | White powder |
| Purity | 99.73% |
| Salt coefficient | / |
| Molecular weight | / |
| Storage condition | -20°C |
| Formulation process | 20% PEG400 + 10% (30% Solutol HS15) + 70% normal saline |

40 mg of the substance to be tested was weighed and put into a brown sample vial, and 0.8 mL of PEG400 was added. The vial was vortexed on a vortex, ultrasonicated for 15 minutes, and heated in a water bath at 40°C for 15 minutes, resulting in a suspension. Then 0.4 mL of 30% Solutol HS15 was added and the vial was vortexed on the vortex until thoroughly mixed. Then 2.8 mL of normal saline was added and the vial was vortexed on the vortex until thoroughly mixed to form a solution, in which the compound concentration was 10 mg/mL. The solution was prepared every three days and stored at 4°C for later use.

### 3. Experimental instruments

Anesthesia machine: Raymain, RM-HSIV-u;
High-speed homogenizer: IKA, T10 basic;
Compound weighing balance: Sartorius, CPA225D;
Animal weighing balance: Changzhou Tianzhiping electronic balance, YH2000.

### 4. Experimental animals and housing environment

| | |
|---|---|
| Animal strain: | DBA/1 mice |
| Supplier: | Beijing Charles River Laboratory Animal Technology Co., Ltd. |
| Sex, body weight: | Male, 14-20 g, specific pathogen free (SPF) |
| Adaptation period: | 3-7 days |
| Environment: | SPF animal breeding rooms, Animal Breeding Center at WuXi AppTec, Waigaoqiao, Shanghai |
| Temperature: | 20-26°C |
| Humidity: | 40-70% |
| Illumination: | Fluorescent lamps, 12 hours of light (08:00-20:00) and 12 hours of dark (20:00-08:00) |
| Housing density: | 5 animals/cage |
| Food: | Free access to food (radiation-sterilized) |
| Drinking water: | Free access to drinking water (prepared by a Molecular (Ultra)pure Water Purifier). |

The animal operations reported and described in this Example was reviewed and approved by the Institutional Animal Care and Use Committee (IACUC) of WuXi AppTec.

### 5. Experimental methods

### 1) Immunization with type II collagen/complete Freund's adjuvant

Preparation of acetic acid: 2 N of acetic acid was diluted to 100 mM, filtered with a 0.22 µm filter membrane, and stored at 4°C.

Bovine type II collagen solution: Bovine type II collagen (CII) was dissolved in 100 mM of acetic acid solution and stored at 4°C overnight. The final concentration of collagen was 8 mg/mL.

Preparation of emulsion: the CII solution stored overnight was mixed with an equal volume of complete Freund's adjuvant, and homogenized on ice using a high-speed homogenizer at 30,000 rpm for approximately 60 minutes until the solution formed a stable emulsion.

### 2) Induction of arthritis

DBA/1 mice were anesthetized with isoflurane and injected subcutaneously with 50 µL of prepared collagen emulsion (containing 200 µg of CII) in the tail. The day of the first immunization was recorded as Day 0, and the subsequent days were marked sequentially. On Day 21, mice were injected with a same volume of collagen emulsion in the tail.

### 3) Dosing and dosage design

When the model mice showed clinical symptoms with an average score of about 0.5 (around Day 28), they were randomly divided into 5 experimental groups of 8 mice in each group according to body weight and score. Dosing lasted for 14 consecutive days. Compounds were prepared every 3 days and stored at 4°C.

**Table 36. Grouping and dosage design**

| Group | Number of animals | Dose of administration (mg/kg) | Route of administration | Frequency of administration | Period of administration |
|---|---|---|---|---|---|
| Normal group | 5 | - | - | - | - |
| Solvent group | 8 | NA | PO | QD | 2 weeks |
| Positive control (Tofacitinib) | 8 | 10 | PO | BID | 2 weeks |
| Nepicastat | 8 | 50 | PO | QD | 2 weeks |

### 4) Determination of arthritis pathogenesis indicators

Since Day 28, mice were weighed three times a week and clinical scores were recorded until the end of the experiment.

Clinical score: scores were given according to different degrees of lesions (redness and swelling, arthrentasis) on a scale of 0-4 points, the highest score was 4 points for each limb and 16 points for each animal.

**Table 37. Clinical scoring criteria for arthritis**

| Score | Clinical symptom |
|---|---|
| 0 | No erythema, redness or swelling |
| 1 | Erythema or mild redness and swelling near the tarsus or on ankle joint or metatarsus, and redness and swelling on one toe |
| 2 | Slight erythema and swelling on ankle joint and metatarsus, or redness and swelling on more than two toes |
| 3 | Moderate erythema and swelling on ankle and wrist joints and metatarsus |
| 4 | Severe redness and swelling on ankle and wrist joints, metatarsus and toes |

### 5) Determination of arthritis pathogenesis indicators

a. Mouse hind paws were collected and fixed in PFA for H&E staining and pathology scoring;
b. Mouse spleen was collected for FACS staining.

### 6) Statistical processing

Experimental data were represented as mean ± standard error. Body weight and clinical score were analyzed by two-way ANOVA, and difference with p < 0.05 was considered as significant.

### 6. Experimental results

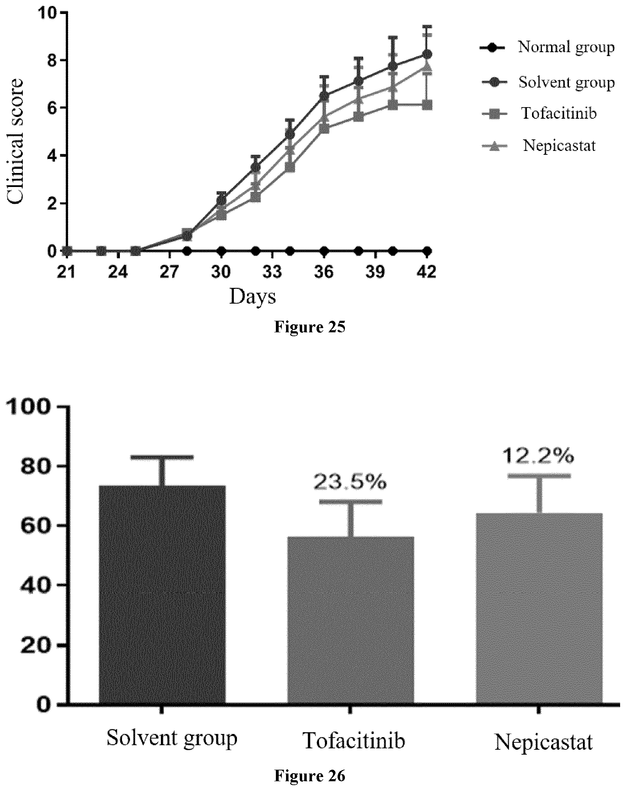

It could be seen from Figures 24 to 27 that Nepicastat had some known effects on collagen-induced arthritis in mice, but not as obvious as the positive control drug.

### Example 10: Therapeutic effect of DBH inhibitors on 2,4-dinitrobenzenesulfonic acid (DNBS)-induced enteritis in rats

### 1. Materials and instruments

**Table 38. Reagents**

| Reagent | Supplier | Catalog No. |
|---|---|---|
| Prednisone acetate tablets (referred to as Prednisone) | Shanghai Sine Pharmaceutical Laboratories Co., Ltd. | 017180604 |
| Sodium carboxymethylcellulose | Sinopharm Chemical Reagent Co., Ltd. | 20180412 |
| Normal saline | Jiangxi Kelun Pharmaceutical Co, Ltd. | C19070906 |
| PEG400 | Sinopharm Chemical Reagent Co., Ltd. | 20190806 |
| 30% Solutol HS15 | Sigma Aldrich | BCCB9630 |
| 2,4-Dinitrobenzenesulfonic acid (DNBS) | Tokyo Chemical Industry (TCI) | LSMRO-RA |
| Ethanol | Sinopharm Chemical Reagent Co., Ltd. | 20190313 |
| Zoletil | Virbac S.A. | N/A |
| Xylazine | Tokyo Chemical Industry (TCI) | X0059 |
| Glucose sodium chloride injection | Shandong Hualu Pharmaceutical Co., Ltd. | G18071503 |

**Table 39. Instruments**

| Instrument | Supplier | Type |
|---|---|---|
| Enema hose | Vygon, France | ACL 7157348 |

### 2. Preparation and storage of drug solution

Substance to be tested - Nepicastat solution: 12 mg of Nepicastat was weighed and put into a brown sample vial, and 0.8 mL of PEG400 was added. The vial was vortexed and ultrasonicated for 15 minutes, and heated in a water bath at 40°C for 15 minutes to form a suspension. Then 0.4 mL of 30% Solutol HS15 was added and the vial was vortexed. Then 2.8 mL of normal saline was added and the vial was vortexed for complete dissolution.

Reference substance - Prednisone solution: Prednisone was used at a concentration of 0.9 mg/mL, and prepared into a suspension using 0.5% sodium carboxymethylcellulose twice a week.

Preparation of DNBS solution: DNBS powder was dissolved in 30% ethanol to a final concentration of 50 mg/mL.

### 3. Handling of rats

Animal species and strains: Wistar rats;
Dosing records: no drug history;
Sex, age, body weight: male, 5 to 6 weeks old, 160 to 180 g;
Breeder/supplier: Shanghai SLAC Laboratory Animal Co., Ltd.
Experimental institution: animal rooms of PharmaLegacy;
Adaptation period: 7 days;
Room: conventional area rooms;
Room temperature: 20 to 26□C;
Indoor relative humidity: 40 to 70%;
Light: fluorescent lamp for illumination, 12 hours with illumination and 12 hours without illumination;
Animal housing: 2 to 4 rats per cage (in the same dosing group);
Food: free access to diet (radiation sterilized, Jiangsu Xietong Pharmaceutical Bioengineering Co., Ltd., China);
Water: free access to drinking water (reverse osmosis treated or autoclaved).

A total of 90 male Wistar rats were purchased from Shanghai SLAC Laboratory Animal Co., Ltd., all of which were specific pathogen free, and were about 4 to 5 weeks old (140 to 150 g) when arrived at the animal rooms of PharmaLegacy.

Upon arrival at PharmaLegacy, the animals were transferred from the transport packages to rat cages and each animal was checked by the staff. The checking included appearance, limbs and cavities, and whether there were abnormalities when the animal stayed still or moved. The adaptation period was 7 days.

The experimental operation protocols designed for application to animals were approved by the IACUC (Institutional Animal Care and Use Committee) of PharmaLegacy.

The 90 animals were randomly grouped on Day -1 (i.e., the day before the experiment) based on animal body weight to ensure that the body weight of each group of animals were similar, so as to reduce bias. Rats were fasted for 40 hours before the experiment and were injected subcutaneously with 5% glucose saline (10 mL/kg, once daily) during fasting.

Experimental grouping:
On Day 1 of the experiment, fasting rats were anesthetized by intraperitoneal injection with Zoletil (25 mg/kg Tiletamine and 25 mg/kg Zolazepam) and 5 mg/kg Xylazine.

In groups G2-G6, a hose was inserted from the anus to left colic flexure (about 8 cm from the anus) and colitis was induced by DNBS enema (0.5 mL/animal) in rats. The normal control group (G1) received 30% ethanol enema by the same process. The head of the animal after enema was lowered for 15 min, and then the animal was kept in Trendelenburg position until waked to avoid backflow of the enema fluid.

**Table 40. Grouping and dosing regimen**

| Group | Test substance | Model | Number of animals | Route of administration | Concentration | Dose of administration | | Dosing regimen |
|---|---|---|---|---|---|---|---|---|
| | | | | | mg/mL | mL/kg | mg/kg | |
| G1 | Normal control group | Ethanol | 15 | PO | N/A | 10 | N/A | QD × 7 |
| G2 | Model control group | DNBS | 15 | PO | N/A | 10 | N/A | QD × 7 |
| G3 | Prednisone | DNBS | 15 | PO | 0.9 | 10 | 9 | QD × 7 |
| G4 | Test group 1# | DNBS | 15 | PO | 0.3 | 10 | 3 | QD × 7 |
| G5 | Test group 2# | DNBS | 15 | PO | 1 | 10 | 10 | QD × 7 |
| G6 | Test group 3# | DNBS | 15 | PO | 3 | 10 | 30 | QD × 7 |
| In the G1 normal control group, 4 h after 30% ethanol enema, rats received 20% PEG400 + 10% (30% Solutol HS15) + 70% normal saline gavage at 10 mL/kg, once a day for 7 consecutive days; | | | | | | | | |
| In the G2 model control group, 4 h after DNBS induction, rats received the solvent 20% PEG400 + 10% (30% Solutol HS15) + 70% normal saline gavage at 10 mL/kg, once a day for 7 consecutive days; | | | | | | | | |
| In the G3 Prednisone group, 4 h after DNBS induction, rats received Prednisone gavage at 9 mg/kg, once a day for 7 consecutive days; | | | | | | | | |
| In the G4 to G6 group of substance to be tested , 4 h after DNBS induction, rats received substance to be tested gavage at different doses, once a day for 7 consecutive days. | | | | | | | | |

### 4. Detection of indicators

(1) Animal body weight: the animal body weight was measured and recorded every day, and the daily activities of animals were observed to record abnormities. The percentage of body weight was calculated according to the following formula: (body weight on Day X - initial weight) / initial weight] × 100%.
(2) Stool score: during the experiment, the stool state of rats was scored daily (0 = normal, 1 = wet/sticky, 2 = loose, 3 = liquid).
(3) Colon observation: after the end of the experiment, all animals were anesthetized with Zoletil (intravenous injection, 25 mg/kg) and the abdominal cavity was opened.

Blood was collected from the abdominal aorta with EDTA anticoagulation (centrifugation conditions: 4°C, 2,000 g, 10 min). After the animals were sacrificed by bloodletting, the colon (from the cecum to the anus) was dissected and the colon length was measured immediately. The colon was longitudinally incised and rinsed until clean. Then the colon weight and ulcer area were recorded, and the colon was photographed as a whole and divided into three parts, among which two parts were stored at -80°C after flash freezing with liquid nitrogen for MPO and cytokine detection, and the other part of colon was fixed with 10% neutral buffered formalin. If the ulcer was irregularly shaped, it could be considered as rectangular, and then its length and width were measured for ulcer area assessment. Ulcer area (cm²) = ulcer length (cm) × ulcer width (cm).

### (4) Pathological analysis of colon tissue

The proximal, ulcer (corresponding sites can be taken, if there was no ulcer) and distal parts of the colon tissue fixed by neutral buffered formalin were embedded with paraffin, sectioned (thickness 5 µm), and histopathological score was given by H&E staining.

**Table 41**

| Inflammatory cell infiltration | | Tissue damage | |
|---|---|---|---|
| 0 | Occasional or no inflammatory cell infiltration | 0 | No damage to mucous membranes |
| 1 | Inflammatory cell infiltration in lamina propria | 1 | Localized crypt lesions |
| 2 | Inflammatory cell infiltration in submucosa | 2 | Erosions or ulcers of mucous membranes |
| 3 | Transmural inflammatory cell penetration | 3 | Extensive damage to submucosa |

### 5. Statistical analysis

Experimental data were represented as mean ± standard error. The data were analyzed by Graphpad Prism using corresponding statistical methods. Difference with p < 0.05 was considered significant.

### 6. Experimental results

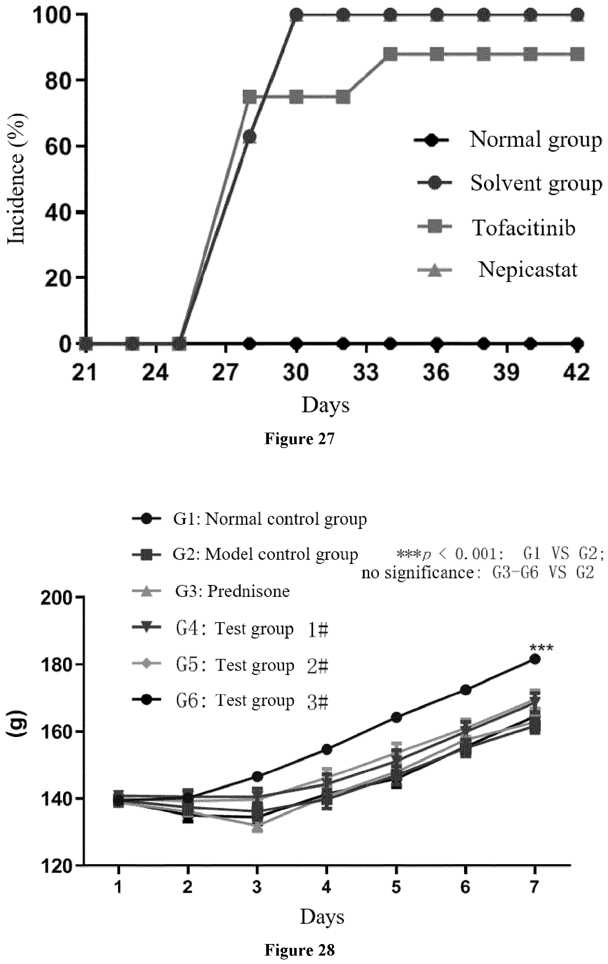

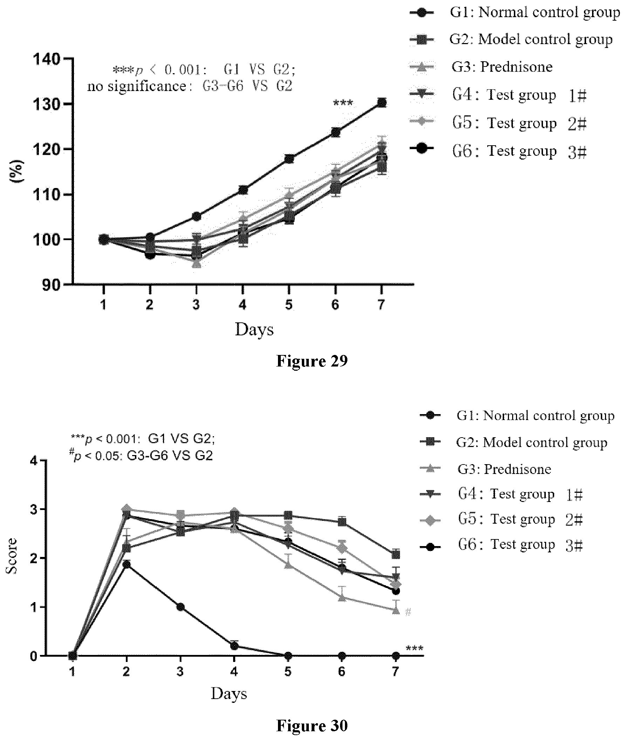

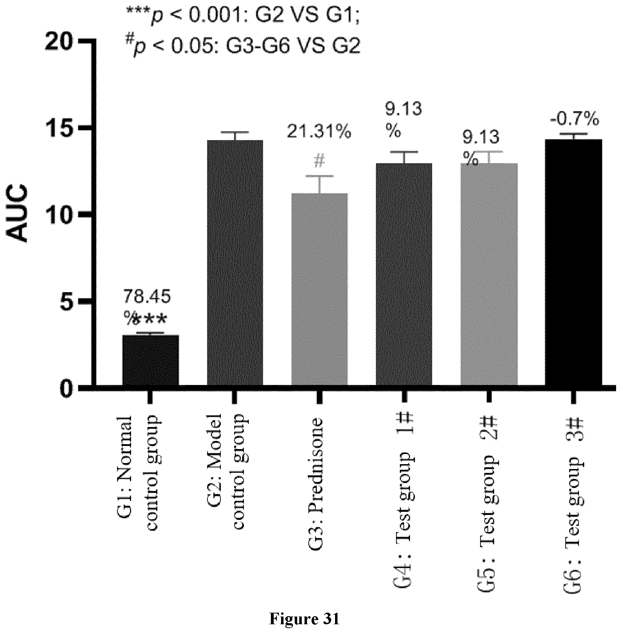

(1) Body weight and stool score were shown in Figures 28 to 31.
(2) Macroscopic evaluation of colon on Day 7

**Table 42. Macroscopic evaluation of colon on Day 7**

| Group | | BW | Colon length | Colon weight | Ulcer length | Ulcer width | Ulcer area |
|---|---|---|---|---|---|---|---|
| | | (g) | (cm) | (g) | (cm) | (cm) | (cm²) |
| G1: Normal control group | Mean | 181.69 | 1.28 | 16.51 | 0.00 | 0.00 | 0.00 |
| | Standard error | 1.05 | 0.03 | 0.24 | 0.00 | 0.00 | 0.00 |
| G2: Model control group | Mean | 161.79 | 2.05 | 10.70 | 1.71 | 1.21 | 2.09 |
| | Standard error | 2.18 | 0.11 | 0.42 | 0.08 | 0.04 | 0.14 |
| G3: Prednisone | Mean | 162.92 | 1.61 | 11.56 | 0.77 | 0.85 | 1.26 |
| | Standard error | 1.67 | 0.08 | 0.39 | 0.21 | 0.29 | 0.62 |
| G4: Test group 1# | Mean | 168.65 | 1.76 | 11.15 | 1.08 | 0.87 | 1.36 |
| | Standard error | 2.91 | 0.09 | 0.36 | 0.21 | 0.15 | 0.33 |
| G5: Test group 2# | Mean | 169.54 | 1.69 | 12.01 | 0.97 | 0.73 | 1.17 |
| | Standard error | 2.80 | 0.09 | 0.40 | 0.22 | 0.17 | 0.32 |
| G6: Test group 3# | Mean | 164.62 | 1.72 | 10.55 | 1.09 | 0.95 | 1.35 |
| | Standard error | 2.06 | 0.08 | 0.23 | 0.20 | 0.15 | 0.30 |

**Table 43. Macroscopic evaluation of colon on Day 7**

| Group | | Ratio | | | | | |
|---|---|---|---|---|---|---|---|
| | | CW/CL/BW | IR1^{a} (%) | CW/BW | IR2^{b} (%) | CW/CL | IR3^{c} (%) |
| | | × 1000 | | × 100 | | × 10 | |
| G1: Normal control group | Mean | 0.43 | / | 0.71 | / | 0.78 | / |
| | Standard error | 0.01 | / | 0.02 | / | 0.02 | / |
| G2: Model control group | Mean | 1.22 | / | 1.28 | / | 1.96 | / |
| | Standard error | 0.09 | / | 0.07 | / | 0.13 | / |
| G3: Prednisone | Mean | 0.87 | 44.20 | 0.99 | 50.13 | 1.41 | 46.51 |
| | Standard error | 0.05 | / | 0.06 | / | 0.08 | / |
| G4: Test group 1# | Mean | 0.96 | 32.98 | 1.05 | 40.24 | 1.60 | 30.82 |
| | Standard error | 0.06 | / | 0.06 | / | 0.09 | / |
| G5: Test group 2# | Mean | 0.86 | 44.95 | 1.01 | 47.06 | 1.44 | 43.80 |
| | Standard error | 0.07 | / | 0.06 | / | 0.10 | / |
| G6: Test group 3# | Mean | 1.00 | 27.12 | 1.05 | 39.85 | 1.64 | 26.68 |
| | Standard error | 0.05 | / | 0.05 | / | 0.08 | / |

| | | | | | | | |
|---|---|---|---|---|---|---|---|
| BW: body weight; CL: colon length; CW: colon weight; ^{a}: IR1 (inhibition rate 1) = {[CW/CL/BW (model group) - CW/CL/BW (test drug group)] / [CW/CL/BW (model group) - CW/CL/BW (normal group)] } × 100%; ^{b}: IR2 (inhibition rate 2) = {[CW/BW (model group) - CW/BW (test drug group)] / [CW/BW (model) - CW/BW (normal group)]} × 100%; ^{c}: IR3 (inhibition rate 3) = {[CW/CL (model group) - CW/CL (test drug group)] / [CW/CL (model group) - CW/CL (normal group)]} × 100%. | | | | | | | |

(3) Pathological score

**Table 44. Pathological score**

| Group | | Proximal end | | Ulcer end | | Distal end | | Total score | |
|---|---|---|---|---|---|---|---|---|---|
| | | Inflam matio n | Dama ge | Inflam matio n | Dama ge | Inflam matio n | Dama ge | Inflam matio n | Dama ge |
| G1: Normal control group | Mean | 0.87 | 0.67 | 1.13 | 0.87 | 0.80 | 0.53 | 2.80 | 2.07 |
| | Standard error | 0.09 | 0.13 | 0.09 | 0.09 | 0.11 | 0.13 | 0.17 | 0.25 |
| G2: Model control group | Mean | 1.87 | 1.07 | 2.87 | 2.73 | 2.27 | 1.67 | 7.00 | 5.47 |
| | Standard error | 0.22 | 0.30 | 0.09 | 0.21 | 0.18 | 0.33 | 0.26 | 0.36 |
| G3: Prednisone | Mean | 1.73 | 0.80 | 2.33 | 1.87 | 2.00 | 1.33 | 6.07 | 4.00 |
| | Standard error | 0.18 | 0.26 | 0.19 | 0.31 | 0.28 | 0.33 | 0.49 | 0.61 |
| G5: Test group 2# | Mean | 1.60 | 0.47 | 2.40 | 2.13 | 2.07 | 0.93 | 6.07 | 3.53 |
| | Standard error | 0.16 | 0.17 | 0.21 | 0.29 | 0.23 | 0.28 | 0.44 | 0.51 |

### 7. Conclusions

Wistar rats were intracolonically perfused with DNBS to induce inflammatory colitis. Colitis was manifested as a significant decrease in animal body weight, a significant increase in stool form score, a significantly reduced colon length, a significant increase in colon weight and the ratios of colon weight: colon length (i.e., CW/CL), colon weight: body weight (i.e., CW/BW) and colon weight: colon length: body weight (i.e., CW/CL/BW), a significant increase in colon ulcer area, and a significant increase in colon inflammatory cell infiltration score and tissue damage score.

In this Example, the positive control drug Prednisone could significantly reduce the stool form score AUC, colon weight, CW/CL, CW/BW, CW/CL/BW and inflammatory cell infiltration score of colon ulcer end of the model rat, in which the inhibition rates of CW/CL/BW, CW/BW and CW/CL reached 44.20%, 50.13% and 46.51%, respectively.

The inhibition rates of CW/CL/BW, CW/BW and CW/CL in Test group 1# (3 mg/kg) were 32.98%, 40.24% and 30.82%, respectively.

Test group 2# (Nepicastat 10 mg/kg) could significantly reduce CW/CL, CW/CL/BW and the total score of colon tissue injury, and the inhibition rates of CW/CL/BW, CW/BW and CW/CL were 44.95%, 47.06% and 43.80%, respectively.

The inhibition rates of CW/CL/BW, CW/BW and CW/CL in Test group 3# (30 mg/kg) were 27.12%, 39.85% and 26.68%, respectively.

In summary, Nepicastat had certain prophylactic and therapeutic effects on DNBS-induced colitis in rats.

### Example 11: Inhibition of DBH inhibitors on dextran sulfate sodium (DSS)-induced colitis

### 1. Experimental materials

**Table 45. Reagents**

| Reagent name | Source | Catalog No. | Batch No. |
|---|---|---|---|
| Tween80 | Sigma | P4780-100ML | BCCB6908 |
| PEG300 | MedChemExpress | HY-Y0873/CS-0015844 | 85948 |
| PEG400 | Sinopharm Chemical Reagent | 30150828 | / |
| Solutol HS 15 | Sigma-Aldrich | 42966-1KG | BCCB9630 |
| 0.9% sodium chloride injection | Double-crane Pharmaceuticals | / | 190507 |
| DMSO | Sigma-Aldrich | D4540-100ML | BCCB6907 |

**Table 46. Substance to be tested**

| Compound name | Source | Catalog No. | Batch No. | Purity |
|---|---|---|---|---|
| DSS | MP Biomedicals | 160110 | S3045 | / |
| Nepicastat | Asieris Pharmaceuticals | | 0002812-058-01 | 99.79% |
| Cyclosporin A (CsA) | MedChemExpress | HY-BO579/CS-2761 | 31755 | / |
| Fusaric acid | MedChemExpress | HY-128483 | 536-69-6 | 98.1% |
| Disulfiram | MedChemExpress | 97-77-8 | 97170 | 98.8% |
| Fumaric acid | MedChemExpress | HY-W015883 | 110-17-8 | 98% |

Experimental animals: strain C57BL/6 mice; source: Zhejiang Charles River Laboratory Animal Technology Co., Ltd.; sex: female. Main instruments: electronic balance: Sartorius, QUINTIX35-1CN.

### 2. Experimental methods

### 2.1 Preparation and storage of compounds

An appropriate amount of Cyclosporine A (CsA) powder was weighed and put into a brown sample vial, an appropriate proportion of 2% DMSO, 30% PEG300, 5% Tween 80 and 63% Saline was added, and the vial was shaken to dissolve the compound. The solution was prepared every day.

An appropriate amount of Nepicastat was weighed and put into a brown sample vial, and an appropriate amount and certain proportion of 20% PEG400, 10% (30% Solutol HS15) and 70% normal saline was sequentially added. The mixture was mixed thoroughly and ultrasonicated to dissolve the compound. The solution was prepared every 3 days to maintain its stability.

An appropriate amount of Fusaric acid was weighed and put into a brown sample vial, and an appropriate amount of normal saline was added. The mixture was ultrasonicated to dissolve the compound. The solution was prepared every seven days.

An appropriate amount of Disulfiram was weighed and put into a brown sample vial, and an appropriate amount and certain proportion of 50% PEG300 and 50% normal saline was sequentially added. The mixture was ultrasonicated to dissolve the compound and to form a suspension. The suspension should be mixed well before dosing, and was prepared every seven days.

An appropriate amount of Fumaric acid was weighed and put into a brown sample vial, and an appropriate amount of normal saline was added. The mixture was heated in a water bath to dissolve the compound. The solution was prepared every day.

**Table 47. Volume of administration and drug stock concentration**

| Group (n=10) | Volume of administration (mL/kg) | Dose of administration (mg/kg) | Stock concentration (mL/mg) |
|---|---|---|---|
| Normal group | - | - | - |
| Solvent group | | | |
| Cyclosporin A | 10 | 50 | 5 |
| Nepicastat | 10 | 50 | 5 |
| Fusaric acid | 10 | 100 | 10 |
| Disulfiram | 10 | 100 | 10 |
| Fumaric acid | 10 | 100 | 10 |

### 2.2 Animal Housing

Seventy 8-week-old female C57BL/6 mice, weighing approximately 20 g, were individually housed in ventilated cages (IVC, 5 mice per cage) at controlled temperature (20±2°C) with a 12/12-hour light/dark cycle. Prior to the experiment, the mice were housed in an SPF grade animal room for three days for adaptation, and free access to adequate water and food during this period.

### 2.3 Grouping and dosing regimen

The experimental mice were housed in an SPF grade animal room for three days for adaptation, and randomly divided into 7 groups (6 model groups and 1 control group) of 10 mice in each group. Dosing of mice started on Day 0 and ended on Day 7.

**Table 48. Animal grouping and dosing regimen**

| Group | Number of animals | Frequency of administration | Route of administration | Period of administration (days) |
|---|---|---|---|---|
| Normal group | 10 | - | - | - |
| Solvent group | 10 | | | |
| Cyclosporin A | 10 | QD | PO | 8 |
| Nepicastat | 10 | QD | PO | 8 |
| Fusaric acid | 10 | BID | PO | 8 |
| Disulfiram | 10 | QD | PO | 8 |
| Fumaric acid | 10 | QD | PO | 8 |

### 2.4 Model Establishment

The mouse enteritis model of this Example was established by DSS induction: mice in the model group were provided with a 3.1% DSS (dextran sulfate sodium, molecular weight 36,000-50,000) aqueous solution from Day 0 to Day 7, and the DSS aqueous solution was replaced with freshly prepared solution every day to prevent degradation. Mice in the normal control group were provided with normal water. The experimental period was 9 days, and the mice were euthanized and samples were collected at the experimental endpoint.

### 2.5 Disease activity (DAI) score

The DAI score was assessed daily. The DAI score consisted of 3 parts: body weight change, stool characteristics, and hematochezia (or occult blood) score, and the specific scoring criteria were as shown in Table 49. The DAI scoring of all mice was done by the same staff throughout the experiment.

**Table 49. DAI scoring criteria**

| Score | Percentage of body weight loss | Stool characteristics | Hematochezia or occult blood |
|---|---|---|---|
| 0 | 0 | Normal | Occult blood negative |
| 1 | 1 to 5% | Soft stools | Occult blood weak positive |
| 2 | 6 to 10% | Loose stool | Occult blood positive |
| 3 | 11 to 20% | Wet and thin stool | Small amount of blood |
| 4 | >20% | Watery stool | Large amount of blood |

### 2.6 Collection of colon tissue

Mice were euthanized at the experimental endpoint. The colon of mice was dissected, and photograph was taken, the length was measured, and the contents were removed before weighing.

### 2.7 Statistical analysis

The data were expressed as mean ± standard error (X ± s). Body weight change and disease activity score were statistically analyzed by two-way ANOVA and comparison between groups were performed by Dunnett's test. Other data were statistically analyzed by one-way ANOVA and comparison between groups were performed by Dunnett's test. All analyses were performed using GraphPad Prism software. * *P* < 0.05, ***P* < 0.01, *** *P* < 0.005, **** *P* < 0.0001 vs. solvent group.

### 3. Experimental results and analysis

The body weight data in each group of mice during the experiment were collected and the corresponding DAI score was assessed. The data were analyzed by two-way ANOVA.

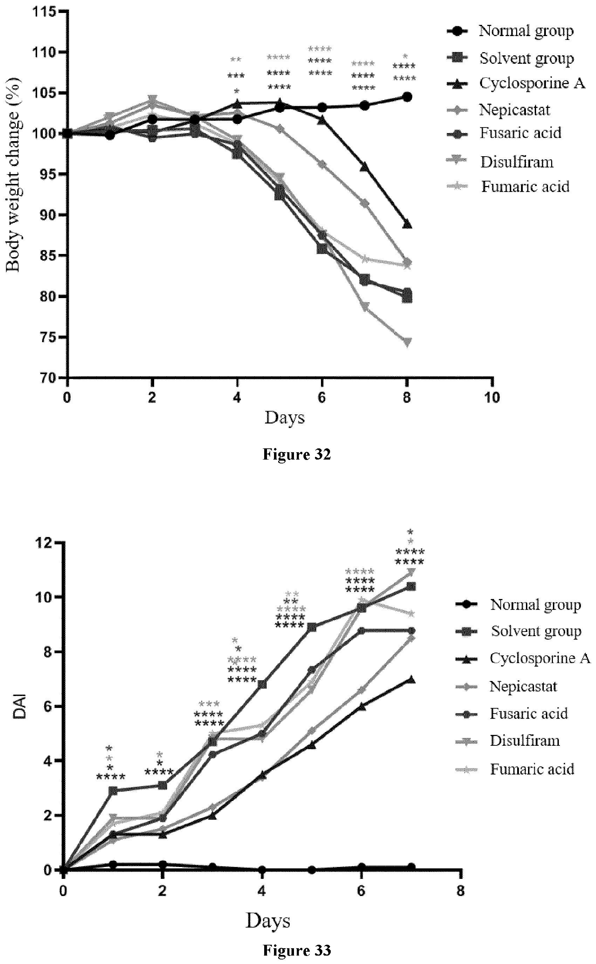

As shown in Figure 32, the positive control CsA (50 mg/kg, QD) and Nepicastat (50 mg/kg, QD) effectively alleviated DSS-induced body weight loss in mice compared with the solvent group. Fusaric acid (100 mg/kg, bid) and Disulfiram (100 mg/kg, qd) failed to effectively alleviate DSS-induced body weight loss in mice compared with the solvent group. Fumaric acid (100 mg/kg, qd) showed a trend of alleviating DSS-induced body weight loss in mice.

As shown in Figure 33, the disease activity score (DAI) of mice in the positive control CsA (50 mg/kg, QD) and Nepicastat (50 mg/kg, QD) groups was significantly lower than that in the solvent group. During administration, the DAI of mice in the Fusaric acid (100 mg/kg, bid), Disulfiram (100 mg/kg, qd) and Fumaric acid (100 mg/kg, qd) groups could be slightly improved compared with the solvent group, among which the performance was relatively obvious on Day 4 and Day 5, but the effect was less than that of Nepicastat.

Considering both the body weight change and the DAI score, it could be seen that the positive control CsA (50 mg/kg, QD) and Nepicastat (50 mg/kg, QD) effectively alleviated the symptoms of body weight loss, diarrhea and hematochezia caused by DSS-induced colitis. The Fusaric acid (100 mg/kg, bid), Disulfiram (100 mg/kg, qd) and Fumaric acid (100 mg/kg, qd) groups could slightly ameliorate the symptoms of diarrhea and hematochezia caused by DSS-induced colitis in mice, but did not effectively alleviate the body weight loss caused by DSS-induced colitis in mice.

### References

1. Rush RA, Geffen LB. Dopamine beta-hydroxylase in health and disease. Critical Reviews in Clinical Laboratory Sciences. 1980, 12(3): 241-77.
2. Glennon RA. Phenylisopropylamine stimulants: amphetamine -related agents. Principles of Medicinal Chemistry (7th ed.). Philadelphia, USA: Wolters Kluwer Health/Lippincott Williams & Wilkins. 2013, pp. 646-648. ISBN 9781609133450.
3. Taylor KB. Dopamine-beta-hydroxylase. Stereochemical course of the reaction. J. Biol. Chem. 1974, 249(2): 454-458.
4. Horwitz D, Alexander RW, Lovenberg W, Keiser HR. Human serum dopamine-β-hydroxylase. Relationship to hypertension and sympathetic activity. Circ. Res. 1973, 32(5): 594-599.
5. Mutschler J, Abbruzzese E, Witt SH, Dirican G, Nieratschker V, Frank J, et al. Functional polymorphism of the dopamine β-hydroxylase gene is associated with increased risk of disulfiram-induced adverse effects in alcohol-dependent patients. Journal of Clinical Psychopharmacology. 2012, 32(4): 578-80.
6. Ella E, Sato N, Nishizawa D, Kageyama S, Yamada H, et al. Association between dopamine beta hydroxylase rs5320 polymorphism and smoking behaviour in elderly Japanese. Journal of Human Genetics. 2012, 57(6): 385-90.
7. Bhaduri N, Sinha S, Chattopadhyay A, Gangopadhyay PK, Singh M, Mukhopadhyay KK. Analysis of polymorphisms in the dopamine beta hydroxylase gene:association with attention deficit hyperactivity disorder in Indian children. Indian Pediatrics. 2005, 42(2):123-9.
8. Cubells JF, Sun X, Li W, Bonsall RW, McGrath JA, Avramopoulos D, Elston RC. Linkage analysis of plasma dopamine β-hydroxylase activity in families of patients with schizophrenia. Human Genetics. 2011, 130(5): 635-43.
9. Combarros O, Warden DR, Hammond N, Cortina-Borja M, Belbin O, Lehmann MG, et al. The dopamine β-hydroxylase -1021C/T polymorphism is associated with the risk of Alzheimer's disease in the Epistasis Project. BMC Medical Genetics. 2010, 11(161): 162.
10. Stanley WC, Li B, Bonhaus DW, Johnson LG, Lee K, Porter S, et al. Catecholamine modulatory effects of nepicastat (RS-25560-197), a novel, potent and selective inhibitor of dopamine-beta-hydroxylase. British Journal of Pharmacology. 1997, 121(8): 1803-9.
11. Beliaev A, Learmonth DA, Soares-da-Silva P. Synthesis and biological evaluation of novel, peripherally selective chromanylimidazolethione-based inhibitors of dopamine beta-hydroxylase. J Med Chem. 2006, 49(3): 1191-7.
12. Cvek B., Dvorak Z. Targeting of Nuclear Factor-κB and Proteasome by Dithiocarbamate Complexes with Metals. Current Pharmaceutical Design. 2007, 13(30): 3155-67.
13. Hegde SS, Friday KF. Dopamine-beta-hydroxylase inhibition: a novel sympatho-modulatory approach for the treatment of congestive heart failure. Current Pharmaceutical Design. 1998, 4(6): 469-79.
14. De La Garza, R 2nd, Bubar MJ, Carbone CL, Moeller FG, Newton TF, et al. Evaluation of the dopamine β-hydroxylase(DBH) inhibitor nepicastat in participants who meet criteria for cocaine use disorder. Prog. Neuropsychopharmacol. Biol. Psychiatry. 2015, 59: 40-8.
15. Nunes T, Rocha JF, Vaz-da-Silva M, Igreja B, Wright LC, et al. Safety, tolerability, and pharmacokinetics of etamicastat, a novel dopamine-β-hydroxylase inhibitor, in a rising multiple-dose study in young healthy subjects. Drugs in R & D. 2010, 10(4): 225-242.
16. Almeida L, Nunes T, Costa R, Rocha JF, Vaz-da-Silva M, Soares-da-Silva P. Etamicastat, a novel dopamine β-hydroxylase inhibitor: tolerability, pharmacokinetics, and pharmacodynamics in patients with hypertension. ClinTher. 2013, 35(12): 1983-96.
17. R C Alaniz, S A Thomas, M Perez-Melgosa, K Mueller, AG Farr, et al. Dopamine beta-hydroxylase deficiency impairs cellular immunity. Proc Natl Acad Sci USA . 1999, 96(5): 2274-8.
18. Gonzalez-Lopez E, Vrana KE. Dopamine beta-hydroxylase and its genetic variants in human health and disease. J Neurochem. 2020, Jan, 152(2): 157-181.
19. Clin Exp Immunol. 2009, May,156(2): 353-362.

## Claims

1. Use of a pathway modulator in the preparation of a medicament; wherein,
the medicament is for the treatment of autoimmune diseases;
the pathway modulator is selected from the group consisting of dopamine β-hydroxylase inhibitor, receptor agonist, receptor antagonist, and the combination thereof.

2. The use according to claim 1, wherein,
the pathway modulator is a dopamine β-hydroxylase inhibitor, and the dopamine β-hydroxylase inhibitor is selected from the group consisting of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof;
preferably, the dopamine β-hydroxylase inhibitor is selected from the group consisting of Nepicastat, Etamicastat, Zamicastat, Fusaric acid, Disulfiram, Fumaric acid, a pharmaceutically acceptable salt thereof, a prodrug thereof and the combination thereof.

3. The use according to claim 1, wherein,
the pathway modulator is Nepicastat or a pharmaceutically acceptable salt thereof, and the unit dose is 10-100 mg/kg, preferably 20-50 mg/kg; or,
the pathway modulator is Etamicastat or a pharmaceutically acceptable salt thereof, and the unit dose is 80-120 mg/kg, preferably 90-110 mg/kg, more preferably 100 mg/kg; or,
the pathway modulator is Zamicastat or a pharmaceutically acceptable salt thereof, and the unit dose is 80-120 mg/kg, preferably 90-110 mg/kg, more preferably 100 mg/kg; or,
the pathway modulator is Fusaric acid or a pharmaceutically acceptable salt thereof, and the unit dose is 80-120 mg/kg, preferably 90-110 mg/kg, more preferably 100 mg/kg; or,
the pathway modulator is Disulfiram or a pharmaceutically acceptable salt thereof, and the unit dose is 80-120 mg/kg, preferably 90-110 mg/kg, more preferably 100 mg/kg; or,
the pathway modulator is Fumaric acid or a pharmaceutically acceptable salt thereof, and the unit dose is 80-120 mg/kg, preferably 90-110 mg/kg, more preferably 100 mg/kg.

4. The use according to any one of claims 1 to 3, wherein,
the autoimmune diseases are selected from the group consisting of Achalasia, Addison's disease, Adult Still's disease, Agammaglobulinemia, Alopecia areata, Amyloidosis, Ankylosing spondylitis, Anti-GBM/Anti-TBM nephritis, Antiphospholipid syndrome, Autoimmune angioedema, Autoimmune dysautonomia, Autoimmune encephalomyelitis, Autoimmune hepatitis, Autoimmune inner ear disease, Autoimmune myocarditis, Autoimmune oophoritis, Autoimmune orchitis, Autoimmune pancreatitis, Autoimmune retinopathy, Autoimmune urticaria, Axonal & neuronal neuropathy, Baló disease, Behcet's disease, Benign mucosal pemphigoid, Bullous pemphigoid, Castleman disease, Celiac disease, Chagas disease, Chronic inflammatory demyelinating polyneuropathy, Chronic recurrent multifocal osteomyelitis, Eosinophilic Granulomatosis, Cicatricial pemphigoid, Cogan's syndrome, Cold agglutinin disease, Congenital heart block, Coxsackie myocarditis, CREST syndrome, Crohn's disease, Dermatitis herpetiformis, Dermatomyositis, neuromyelitis optica, Discoid lupus, Dressler's syndrome, Endometriosis, Eosinophilic esophagitis, Eosinophilic fasciitis, Erythema nodosum, Essential mixed cryoglobulinemia, Evans syndrome, Fibromyalgia, Fibrosing alveolitis, Giant cell arteritis, Giant cell myocarditis, Glomerulonephritis, Goodpasture's syndrome, Granulomatosis with Polyangiitis, Graves'disease, Guillain-Barre syndrome, Hashimoto's thyroiditis, Hemolytic anemia, Henoch-Schonlein purpura, Herpes gestationis or pemphigoid gestationis, Hidradenitis Suppurativa, Hypogammalglobulinemia, IgA Nephropathy, IgG4-related sclerosing disease, Immune thrombocytopenic purpura, Inclusion body myositis, Interstitial cystitis, Juvenile arthritis, Juvenile diabetes Type 1, Juvenile myositis, Kawasaki disease, Lambert-Eaton syndrome, Leukocytoclastic vasculitis, Lichen planus, Lichen sclerosus, Ligneous conjunctivitis, Linear IgA disease, Lupus, Lyme disease chronic, Meniere's disease, Microscopic polyangiitis, Mixed connective tissue disease, Mooren's ulcer, Mucha-Habermann disease, Multifocal Motor Neuropathy , Multiple sclerosis, Myasthenia gravis, Myositis, Narcolepsy, Neonatal Lupus, Neutropenia, Ocular cicatricial pemphigoid, Optic neuritis, Palindromic rheumatism, PANDAS, Paraneoplastic cerebellar degeneration, Paroxysmal nocturnal hemoglobinuria, Parry Romberg syndrome, Pars planitis, Parsonage-Turner syndrome, Pemphigus, Peripheral neuropathy, Perivenous encephalomyelitis, Pernicious anemia, POEMS syndrome, Polyarteritis nodosa, Polyglandular syndromes type I, Polyglandular syndromes type II, Polyglandular syndromes type III, Polymyalgia rheumatica, Polymyositis, Postmyocardial infarction syndrome, Postpericardiotomy syndrome, Primary biliary cirrhosis, Primary sclerosing cholangitis, Progesterone dermatitis, Psoriasis, Psoriatic arthritis, Pure red cell aplasia, Pyoderma gangrenosum, Raynaud's phenomenon, Reactive Arthritis, Reflex sympathetic dystrophy, Relapsing polychondritis, Restless legs syndrome, Retroperitoneal fibrosis, Rheumatic fever, Rheumatoid arthritis, Sarcoidosis, Schmidt syndrome, Scleritis, Scleroderma, Sjögren's syndrome, Sperm & testicular autoimmunity, Stiff person syndrome, Subacute bacterial endocarditis, Susac's syndrome, Sympathetic ophthalmia, Takayasu's arteritis, Temporal arteritis/Giant cell arteritis, Thrombocytopenic purpura, Thyroid eye disease, Tolosa-Hunt syndrome, Transverse myelitis, Type 1 diabetes, Ulcerative colitis, Undifferentiated connective tissue disease, Uveitis, Vasculitis, Vitiligo and Vogt-Koyanagi-Harada Disease;
preferably selected from the group consisting of: Autoimmune colitis, Neuromyelitis optica, Rheumatoid arthritis, Scleroderma, Psoriasis, Uveitis;
the autoimmune colitis is selected from the group consisting of: Crohn's disease, Ulcerative colitis.

5. A method for the treatment of autoimmune diseases, including the following steps:
administering a therapeutically effective amount of pathway modulator to a subject;
wherein, the pathway modulator is selected from the group consisting of dopamine β-hydroxylase inhibitor, receptor agonist, receptor antagonist, and the combination thereof.

6. The method according to claim 5, wherein,
the pathway modulator is a dopamine β-hydroxylase inhibitor, and the dopamine β-hydroxylase inhibitor is selected from the group consisting of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof;
preferably, the dopamine β-hydroxylase inhibitor is selected from the group consisting of Nepicastat, Etamicastat, Zamicastat, Fusaric acid, Disulfiram, Fumaric acid, a pharmaceutically acceptable salt thereof, a prodrug thereof and the combination thereof.

7. The method according to claim 5 or 6, wherein,
the autoimmune diseases are as defined in claim 4.

8. A pharmaceutical composition for the treatment of autoimmune diseases, comprising:
a pathway modulator, and
a pharmaceutically acceptable carrier;
wherein, the pathway modulator is selected from the group consisting of dopamine β-hydroxylase inhibitor, receptor agonist, receptor antagonist, and the combination thereof.

9. The pharmaceutical composition according to claim 8, wherein,
the pathway modulator is a dopamine β-hydroxylase inhibitor, and the dopamine β-hydroxylase inhibitor is selected from the group consisting of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof;
preferably, the dopamine β-hydroxylase inhibitor is selected from the group consisting of Nepicastat, Etamicastat, Zamicastat, Fusaric acid, Disulfiram, Fumaric acid, a pharmaceutically acceptable salt thereof, a prodrug thereof and the combination thereof.

10. The pharmaceutical composition according to claim 8, wherein, the autoimmune diseases are as defined in claim 4.

11. The use according to claim 1, wherein the medicament is used for one or more selected from the group consisting of:
reducing the proportion of CD4+ T cells, increasing the proportion of regulatory T cells, increasing the proportion of CD8+ T cells, reducing the secretion of pro-inflammatory factors of CD4+ T cells, reducing the secretion of pro-inflammatory factors of CD8+ T cells, inhibiting the activation of B cells and inhibiting the activation of NK cells; reducing the content of lymphocytes, neutrophils and monocytes in the peripheral blood; reducing inflammatory cell infiltration and subdermal capillary hyperplasia in the dermis layer; ameliorating skin fibrosis; reducing the incidence of uveitis; ameliorating skin inflammation; improving stool form score, improving CW/CL, improving CW/BW, improving CW/CL/BW, inhibiting the increase of colon ulcer area, improving colon inflammatory cell infiltration score, improving tissue damage score; improving disease activity score, ameliorating hematochezia or occult blood.

12. The use according to claim 11, wherein:
the pro-inflammatory factors of CD4+ T cells are one or more of IL-17A, IFN-γ and TNF-α;
the pro-inflammatory factors of CD8+ T cells are IL-17A and/or TNF-α.

13. The use according to claim 11, wherein, the regulatory T cells are CD25+FOXP3+ Treg cells;
and/or, the B cells are B220+ cells, preferably CD69+B220+ B cells;
and/or, the NK cells are NK1.1+ cells, preferably NK1.1+CD107a+ NK cells.

14. A method for the regulation of immune cell functions *in vivo* or *in vitro,* including the following steps:
contacting an effective amount of pathway regulator with immune cells *in vivo* or *in vitro,*
wherein,
the immune cells are from a subject;
the pathway modulator is selected from the group consisting of dopamine β-hydroxylase inhibitor, receptor agonist, receptor antagonist, and the combination thereof;
the regulation of immune cell functions refers to one or more functions selected from the group consisting of : reducing the proportion of CD4+ T cells, increasing the proportion of regulatory T cells, increasing the proportion of CD8+ T cells, reducing the secretion of pro-inflammatory factors of CD4+ T cells, reducing the secretion of pro-inflammatory factors of CD8+ T cells, inhibiting the activation of B cells and inhibiting the activation of NK cells.

15. The method according to claim 14, wherein, the dopamine β-hydroxylase inhibitor is selected from the group consisting of Nepicastat, Etamicastat, Zamicastat, Fusaric acid, Disulfiram, Cysteamine, Pantethine, Copper chelating agent, Fumaric acid, Hydralazine, 2-Thiophen-2-ylallylamine, a pharmaceutically acceptable salt thereof, a prodrug thereof and the combination thereof;
preferably, the dopamine β-hydroxylase inhibitor is selected from the group consisting of Nepicastat, Etamicastat, Zamicastat, Fusaric acid, Disulfiram, Fumaric acid, a pharmaceutically acceptable salt thereof, a prodrug thereof and the combination thereof.

16. The method according to claim 14, wherein:
the pro-inflammatory factors of CD4+ T cells are one or more of IL-17A, IFN-γ and TNF-α;
the pro-inflammatory factors of CD8+ T cells are IL-17A and/or TNF-α.

17. The method according to claim 14, wherein, the regulatory T cells are CD25+FOXP3+ Treg cells;
and/or, the B cells are B220+ cells, preferably CD69+B220+ B cells;
and/or, the NK cells are NK1.1+ cells, preferably NK1.1+CD107a+ NK cells.
